# EUROPEAN PATENT APPLICATION

(11) **EP 4 653 487 A1**
(43) Date of publication of application: **26.11.2025**
(21) Application number: 24750394.9
(22) Date of filing: 01.02.2024
(51) Int. Cl.: C08G 81/00, C07K 1/02, C07K 14/435, C08G 75/045, C08L 67/00, C08L 69/00, C08L 71/00, C08L 77/04, D01F 4/02, D01F 6/92, D01F 6/94

(54) **POLYMER COMPOUND PRODUCTION METHOD**

(30) Priority: 03.02.2023 JP 2023015616; 03.04.2023 JP 2023059954
(71) Applicant: Spiber Inc., Tsuruoka-shi, Yamagata 997-0052 (JP)
(72) Inventor: SAKATA Kazuki, Tsuruoka-shi, Yamagata 997-0052 (JP); TAKAMI Taku, Tsuruoka-shi, Yamagata 997-0052 (JP); NIWA Takahiro, Tsuruoka-shi, Yamagata 997-0052 (JP); KAGATA Hideki, Tsuruoka-shi, Yamagata 997-0052 (JP)
(74) Representative: Handley, Matthew Edward
(86) International application number: PCT/JP2024/003344
(87) International publication number: WO 2024/162445

(57) **Abstract**

One aspect of the present disclosure provides a polymer compound production method including subjecting a polypeptide having at least one mercapto group and at least one compound selected from the group consisting of a polyether, a polyester, and a polycarbonate and having two structures represented by the following general formula (1) to a heating reaction in dimethyl sulfoxide in the presence of a base and a reducing agent. 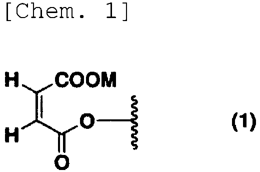 [In general formula (1), M represents any one of H, Na, K, NHEt₃, and NHEtiPr₂]

## Description

### Technical Field

The present disclosure relates to a polymer compound production method, a method for producing a polymer compound solution, a method for producing a film, a method for producing a fiber, a film, and a fiber.

### Background Art

A structural protein such as a silk fibroin or a spider silk fibroin is attracting attention as an alternative to a structural material made of a synthetic resin and the like because the structural protein can exhibit excellent robustness. In recent years, development of recombinant technology has led to development of mass production technology of a recombinant structural protein imitating the above-described structural protein (for example, Patent Literature 1).

Since a protein has many functional groups capable of hydrogen bonding, such as an amide group in a main chain and a carboxy group, an amino group, a hydroxy group, and a mercapto group in a side chain, a large number of hydrogen bonds are formed between molecules. For this reason, it is considered that a molded body obtained by subjecting a structural protein to heat-and-pressure molding has excellent mechanical strength, but the molded body also has a brittle property in which cracking or rupture is caused by receiving an impact or the like. The above-described circumstance is an obstacle to using a protein as an alternative to a common general-purpose plastic.

As a means for modifying a protein, a method for binding a compound having a property different from that of a protein to the protein has been studied. For example, Patent Literature 2 discloses a method for producing a protein material, the method including a step of binding a substance having excellent water resistance to a predetermined modified fibroin.

Patent Literature 3 discloses, for example, a polymer production method in which a thioether bond is generated using a 1,4-addition reaction (Michael addition reaction) between a mercapto group of cysteine constituting a polypeptide skeleton and a carbon-carbon double bond of maleimide or a maleic acid derivative to introduce at least one functional group selected from the group consisting of a polyether group, a polyester group, a polycarbonate group, a polyamide group, a polyol group, and a modified polysaccharide group into the polypeptide skeleton.

### Citation List

### Patent Literature

Patent Literature 1: WO 2015/178466 A
Patent Literature 2: WO 2019/194146 A
Patent Literature 3: WO 2021/187502 A
Patent Literature 4: JP 05-336963 A
Patent Literature 5: WO 1994/023021 A
Patent Literature 6: WO 2019/194263 A

### Summary of Invention

### Technical Problem

Although the production method described in Patent Literature 3 is useful in that various functional groups can be introduced into the polypeptide skeleton, it is necessary to more strictly control the reaction, reaction efficiency, and the like depending on a structure of a Michael addition receptor capable of performing the Michael addition reaction with a mercapto group of the polypeptide skeleton, and thus there is room for improvement as a technique for synthesizing raw materials for industrial materials.

For example, when a maleimide group is used as the Michael addition receptor, not only a designed molecule but also a polymer compound having unexpected long chain growth, branching, or the like may be generated due to high reactivity of a compound to be the Michael addition receptor, viscosity of a reaction solution may increase due to the generation of the polymer compound, and furthermore, control of the reaction system may be difficult. In such a situation, a ratio at which the raw material polypeptide becomes a desired polymer compound is low, and a raw material conversion ratio tends to decrease. Furthermore, it is difficult to sufficiently control the reaction system by adjusting reaction conditions such as acidity of a reaction solution, reaction temperature, and a stirring rate.

Therefore, it is conceivable to adopt a less reactive Michael addition receptor itself as a reactive substrate. However, a decrease in raw material conversion ratio may occur due to a decrease in reactivity of the substrate itself.

An object of the present disclosure is to provide a production method capable of increasing a raw material conversion ratio as compared with a conventional method in the above-described production of a polymer compound.

### Solution to Problem

According to study of the present inventors, in a case where a conventional production method is used on an industrial scale, when a reaction of introducing a predetermined functional group into a polypeptide is performed using dimethyl sulfoxide which is an aprotic polar solvent as a solvent, formation of a disulfide bond between mercapto groups of the polypeptide is promoted by heating at the time of synthesis, and thus it has been found that it may be difficult to bind the functional group to the polypeptide by the Michael addition reaction using the mercapto group. When a rate of the Michael addition reaction is sufficiently faster than that of a disulfide bond formation reaction, it is estimated that this does not cause a big problem. However, in practice, for example, in a reaction between a substrate having a (meth)acryloyloxy group or the like and a polypeptide, it has been confirmed that the rate of the Michael addition reaction is slow and a reaction of forming the disulfide bond between the polypeptides is promoted, and thus synthesis of a target polymer compound does not sufficiently proceed. The present disclosure has been made based on the above-described finding.

The present invention provides the following [1] to [34].
[1] A polymer compound production method including subjecting
   a polypeptide having at least one mercapto group, and
   at least one compound selected from the group consisting of a polyether, a polyester, and a polycarbonate and having two structures represented by the following general formula (1): 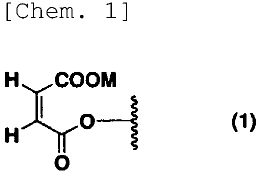
   [In general formula (1), M represents any one of H, Na, K, NHEt₃, and NHEtiPr₂]
   to a heating reaction in dimethyl sulfoxide in the presence of a base and a reducing agent.
[2] A polymer compound production method including subjecting
   a polypeptide having at least one mercapto group, and
   at least one compound selected from the group consisting of a polyether, a polyester, and a polycarbonate and having two structures represented by the following general formula (2): 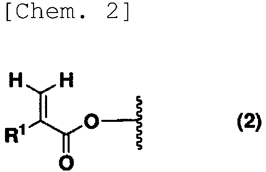
   [In general formula (2), R¹ represents a hydrogen atom or a methyl group]
   to a heating reaction in dimethyl sulfoxide in the presence of a base, a reducing agent, and a polymerization inhibitor.
[3] A polymer compound production method including subjecting
   a polypeptide having at least one mercapto group, and
   at least one compound selected from the group consisting of a polyether, a polyester, and a polycarbonate and having two structures represented by the following general formula (1): 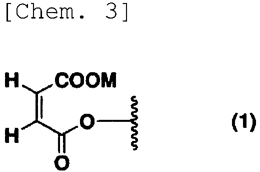
   [In general formula (1), M represents any one of H, Na, K, NHEt₃, and NHEtiPr₂]
   to a heating reaction in dimethyl sulfoxide in the presence of a reducing agent.
[4] A polymer compound production method including subjecting
   a polypeptide having at least one mercapto group, and
   at least one compound selected from the group consisting of a polyether, a polyester, and a polycarbonate and having two structures represented by the following general formula (2): 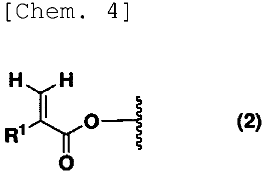
   [In general formula (2), R¹ represents a hydrogen atom or a methyl group]
   to a heating reaction in dimethyl sulfoxide in the presence of a reducing agent and a polymerization inhibitor.
[5] The polymer compound production method according to any one of [1] to [4], wherein the reducing agent is at least one selected from the group consisting of a dithiol, sodium sulfate, sodium sulfite, and sodium dithionite.
[6] The production method according to any one of [1] to [5], wherein a blending amount of the reducing agent is 0.5 equivalents or more with respect to a mercapto group of the polypeptide.
[7] The production method according to any one of [1] to [6], wherein the reaction between the polypeptide and the compound is performed at 50°C or higher.
[8] A method for producing a polymer compound solution, the method including isolating a polymer compound obtained by the production method according to any one of [1] to [7], and then dissolving the polymer compound in a solvent.
[9] A method for producing a film, the method including molding a film from a polymer compound solution obtained by the production method according to [8].
[10] A method for producing a fiber, the method including spinning a polymer compound solution obtained by the production method according to [8].
[11] A method for producing a film, the method including: subjecting a polypeptide having at least one mercapto group and at least one compound selected from the group consisting of a polyether, a polyester, and a polycarbonate and having two structures represented by the following general formula (1) to a heating reaction in dimethyl sulfoxide in the presence of a base and a reducing agent to obtain a polymer compound solution; and
   molding a film from the polymer compound solution. 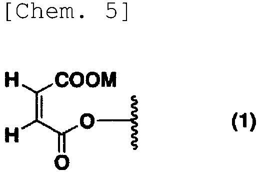
   [In general formula (1), M represents any one of H, Na, K, NHEt₃, and NHEtiPr₂]
[12] A method for producing a film, the method including:
   subjecting a polypeptide having at least one mercapto group and at least one compound selected from the group consisting of a polyether, a polyester, and a polycarbonate and having two structures represented by the following general formula (2) to a heating reaction in dimethyl sulfoxide in the presence of a base, a reducing agent, and a polymerization inhibitor; and
   molding a film from the polymer compound solution. 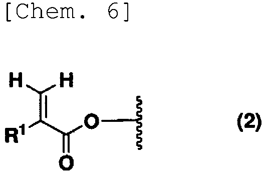
   [In general formula (2), R¹ represents a hydrogen atom or a methyl group]
[13] A method for producing a fiber, the method including: subjecting a polypeptide having at least one mercapto group and at least one compound selected from the group consisting of a polyether, a polyester, and a polycarbonate and having two structures represented by the following general formula (1) to a heating reaction in dimethyl sulfoxide in the presence of a base and a reducing agent to obtain a polymer compound solution; and
   spinning the polymer compound solution. 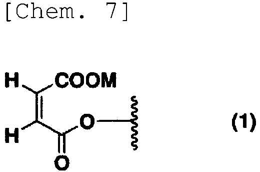
   [In general formula (1), M represents any one of H, Na, K, NHEt₃, and NHEtiPr₂]
[14] A method for producing a fiber, the method including:
   subjecting a polypeptide having at least one mercapto group and at least one compound selected from the group consisting of a polyether, a polyester, and a polycarbonate and having two structures represented by the following general formula (2) to a heating reaction in dimethyl sulfoxide in the presence of a base, a reducing agent, and a polymerization inhibitor; and
   spinning the polymer compound solution. 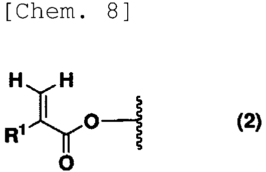
   [In general formula (2), R¹ represents a hydrogen atom or a methyl group]
[15] A film containing a polymer compound, wherein
   the polymer compound has
   a structure in which a polypeptide moiety and
   at least one structural unit selected from the group consisting of a polyether structure, a polyester structure, and a polycarbonate structure directly bind to each other by a structure represented by the following general formula (3), (4), or (5) such that the polypeptide moiety binds to S of the following general formula (3), (4), or (5), 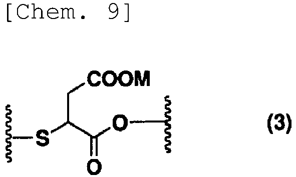 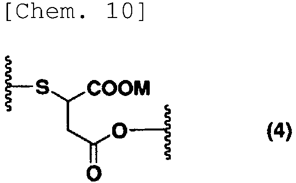 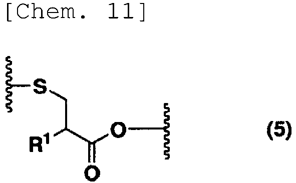
   [In general formulas (3) and (4), M represents any one of H, Na, K, NHEt₃, and NHEtiPr₂, and in general formula (5), R¹ represents H or Me], and
   the film has an elongation of 400% or more and breaking strength higher than yield point strength.
[16] A fiber containing a polymer compound, wherein
   the polymer compound has
   a structure in which a polypeptide moiety and
   at least one structural unit selected from the group consisting of a polyether structure, a polyester structure, and a polycarbonate structure directly bind to each other by a structure represented by the following general formula (3), (4), or (5) such that the polypeptide moiety binds to S of the following general formula (3), (4), or (5), 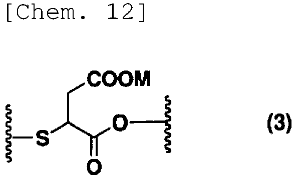 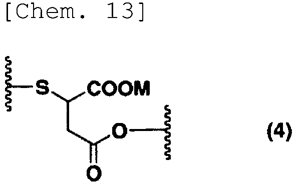 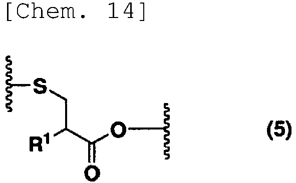
   [In general formulas (3) and (4), M represents any one of H, Na, K, NHEt₃, and NHEtiPr₂, and in general formula (5), R¹ represents H or Me], and
   the fiber has an elongation of 400% or more and breaking strength higher than yield point strength.
[17] A fiber containing a synthetic polymer obtained by the production method according to any one of [1] to [8], and having an elongation of 400% or more and a ratio of breaking strength to yield point strength of more than 1.
[18] A film containing a synthetic polymer obtained by the production method according to any one of [1] to [8], and having an elongation of 400% or more and a ratio of breaking strength to yield point strength of more than 1.
[19] A method for producing a solution-state adhesive, the method including a step of dissolving a polymer compound obtained by the method according to any one of [1] to [8] in a solvent.
[20] A method for producing a water-dispersible adhesive, the method including a step of dispersing a polymer compound obtained by the method according to any one of [1] to [8] in an aqueous medium.
[21] A method for producing a film-shaped adhesive, the method including a step of molding a film from a solution in which a polymer compound obtained by the method according to any one of [1] to [8] is dissolved.
[22] A method for producing a powdery adhesive, the method including a step of obtaining a powder composition containing a polymer compound obtained by the method according to any one of [1] to [8].
[23] A method for producing an adhesive body, the method including interposing a solution obtained by dissolving a polymer compound obtained by the method according to any one of [1] to [8] in a solvent between a plurality of adherends, and then removing the solvent from the solution to solidify the polymer compound, thereby bonding the adherends to each other.
[24] A method for producing an adhesive body, the method including interposing an aqueous dispersion obtained by dispersing a polymer compound obtained by the method according to any one of [1] to [8] in an aqueous medium between a plurality of adherends, and then removing the aqueous medium from the aqueous dispersion to solidify the polymer compound, thereby bonding the adherends to each other.
[25] A method for producing an adhesive body, the method including softening a film containing a polymer compound obtained by the method according to any one of [1] to [8] by swelling or heating, interposing the film between a plurality of adherends, and then curing the film in a state of being brought into pressure contact with the adherends, thereby bonding the adherends to each other.
[26] A method for producing an adhesive body, the method including heating a powder composition containing a polymer compound obtained by the method according to any one of [1] to [8] in a state where the powder composition is interposed between a plurality of adherends, and pressurizing the powder composition via the adherends to solidify the powder composition, thereby bonding the adherends to each other.
[27] A method for producing a solution-state coating agent, the method including a step of dissolving a polymer compound obtained by the method according to any one of [1] to [8] in a solvent.
[28] A method for producing a water-dispersible coating agent, the method including a step of dispersing a polymer compound obtained by the method according to any one of [1] to [8] in an aqueous medium.
[29] A method for producing a film-shaped coating agent, the method including a step of molding a film from a solution in which a polymer compound obtained by the method according to any one of [1] to [8] is dissolved.
[30] A method for producing a powdery coating agent, the method including a step of obtaining a powder composition containing a polymer compound obtained by the method according to any one of [1] to [8].
[31] A method for producing a laminate including a base material and a coating layer laminated on at least a part of a surface of the base material, the method including
   supplying a solution obtained by dissolving a polymer compound obtained by the method according to any one of [1] to [8] in a solvent to at least a part of the surface of the base material to coat at least a part of the surface of the base material with the solution, and then removing the solvent from the solution to solidify the polymer compound, thereby laminating the coating layer on at least a part of the surface of the base material.
[32] A method for producing a laminate including a base material and a coating layer laminated on at least a part of a surface of the base material, the method including
   supplying an aqueous dispersion obtained by dispersing a polymer compound obtained by the method according to any one of [1] to [8] in an aqueous medium to at least a part of the surface of the base material to coat at least a part of the surface of the base material with the aqueous dispersion, and then removing the aqueous medium from the aqueous dispersion to solidify the polymer compound, thereby laminating the coating layer on at least a part of the surface of the base material.
[33] A method for producing a laminate including a base material and a coating layer laminated on at least a part of a surface of the base material, the method including
   softening a film containing a polymer compound obtained by the method according to any one of [1] to [8] by swelling or heating, placing the film on at least a part of the surface of the base material, and then curing the film in a state of being brought into pressure contact with the base material, thereby laminating the coating layer on at least a part of the surface of the base material.
[34] A method for producing a laminate including a base material and a coating layer laminated on at least a part of a surface of the base material, the method including
   heating a powder composition containing a polymer compound obtained by the method according to any one of [1] to [8] in a state where the powder composition is placed on at least a part of the surface of the base material, and pressurizing the powder composition between a pressurizing body and the base material to solidify the powder composition, thereby laminating the coating layer on at least a part of the surface of the base material.

One aspect of the present disclosure provides a polymer compound production method including subjecting a polypeptide having at least one mercapto group and at least one compound selected from the group consisting of a polyether, a polyester, and a polycarbonate and having two structures represented by the following general formula (1) to a heating reaction in dimethyl sulfoxide in the presence of a base and a reducing agent. 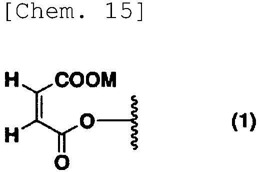 [In general formula (1), M represents any one of H, Na, K, NHEt₃, and NHEtiPr₂]

In one example of the above production method, a method is adopted in which a base and a reducing agent are present in a reaction system when a polypeptide and a predetermined compound of the present disclosure are caused to react with each other. The reducing agent itself is stable in dimethyl sulfoxide, hardly causes the Michael addition reaction with the functional group represented by formula (1), and can inhibit formation of a disulfide bond between mercapto groups of the polypeptide. The base can activate a mercapto group of the polypeptide to promote the Michael addition reaction with the functional group represented by formula (1). By such an action, also by a method for performing heating in dimethyl sulfoxide, a skeleton such as a polyether can be introduced into the polypeptide to produce a polymer compound with higher efficiency. In a case of production conditions in which other conditions are common, a desired effect of the present disclosure can be obtained at a higher level by blending the base, but the base can be omitted depending on a level of the desired effect.

One aspect of the present disclosure provides a polymer compound production method including subjecting a polypeptide having at least one mercapto group and at least one compound selected from the group consisting of a polyether, a polyester, and a polycarbonate and having two structures represented by the following general formula (2) to a heating reaction in dimethyl sulfoxide in the presence of a base, a reducing agent, and a polymerization inhibitor. 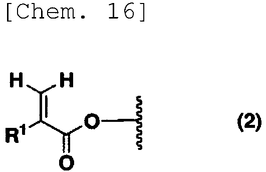 [In general formula (2), R¹ represents a hydrogen atom or a methyl group]

Also in the above example of the production method, a method is adopted in which a base and a reducing agent are present in a reaction system when a polypeptide and a predetermined compound of the present disclosure are caused to react with each other. Here, in the functional group represented by general formula (2), radical polymerization is initiated by being heated in dimethyl sulfoxide, and self-polymerization of at least one compound selected from the group consisting of a polyether, a polyester, and a polycarbonate and having two structures represented by the general formula (2) can occur. However, according to the production method of the present disclosure, a radical polymerization reaction of the functional group represented by general formula (2) can be suppressed by also allowing a polymerization inhibitor to be present in the reaction system. By such an action, also by a method for performing heating in dimethyl sulfoxide, a skeleton such as a polyether can be introduced into the polypeptide to produce a polymer compound with higher efficiency. In a case of production conditions in which other conditions are common, a desired effect of the present disclosure can be obtained at a higher level by blending the base, but the base can be omitted depending on a level of the desired effect.

In the above-described production method, the reducing agent may be at least one selected from the group consisting of a dithiol, sodium sulfate, sodium sulfite, and sodium dithionite. Such a specific reducing agent has high stability in dimethyl sulfoxide, and can further inhibit formation of a disulfide bond between mercapto groups of the polypeptide.

In the production method, the blending amount of the reducing agent may be 0.5 equivalents or more with respect to a mercapto group of the polypeptide. By setting the blending amount of the reducing agent within the above range, formation of a disulfide bond by a reaction between mercapto groups of the polypeptide can be more sufficiently inhibited. This can further improve production efficiency of a target synthetic polymer.

In the production method, the reaction between the polypeptide and the compound may be performed at 50°C or higher.

One aspect of the present disclosure provides a method for producing a polymer compound solution, the method including dissolving a polymer compound obtained by the above-described production method in a solvent.

A method for producing a polymer film, the method including molding a film from a polymer compound solution obtained by the above-described method for producing a polymer compound solution. The molding of the film may be, for example, cast molding.

One aspect of the present disclosure provides a method for producing a polymer fiber, the method including spinning a polymer compound solution obtained by the above-described production method.

One aspect of the present disclosure provides a film containing a polymer compound, in which the polymer compound has a structure in which a polypeptide moiety and at least one structural unit selected from the group consisting of a polyether structure, a polyester structure, and a polycarbonate structure directly bind to each other by a structure represented by the following formula (3), (4), or (5) such that the polypeptide moiety binds to S of the following general formula (3), (4), or (5), and the film has an elongation of 400% or more and breaking strength higher than yield point strength. In general formulas (3) and (4), M represents any one of H, Na, K, NHEt₃, and NHEtiPr₂, and in general formula (5), R1 represents H or Me.

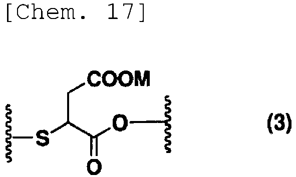

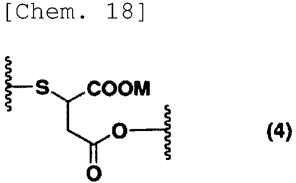

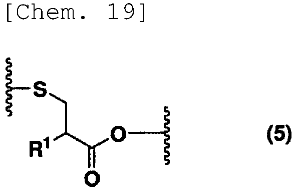

One aspect of the present disclosure provides a fiber containing a polymer compound, in which the polymer compound has a structure in which a polypeptide moiety and at least one structural unit selected from the group consisting of a polyether structure, a polyester structure, and a polycarbonate structure directly bind to each other by a structure represented by the following general formula (3), (4), or (5) such that the polypeptide moiety binds to S of the following general formula (3), (4), or (5), and the fiber has an elongation of 400% or more and breaking strength higher than yield point strength. In general formulas (3) and (4), M represents any one of H, Na, K, NHEt₃, and NHEtiPr₂, and in general formula (5), R1 represents H or Me.

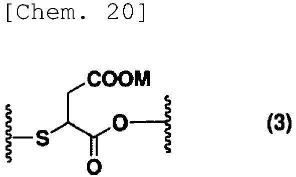

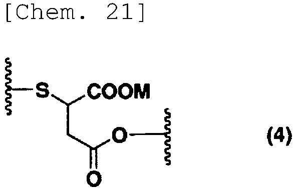

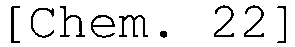

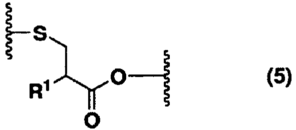

### Advantageous Effects of Invention

According to the present disclosure, it is possible to provide a production method capable of increasing a raw material conversion ratio as compared with a conventional method in the above-described production of a polymer compound.

### Description of Embodiments

Materials exemplified in the present specification can be used singly or in combination of two or more thereof unless otherwise specified. When a plurality of substances corresponding to each component in a composition is present, the content of each component in the composition means the total amount of the plurality of substances present in the composition unless otherwise specified.

In the present specification, a molding material refers to a material used for producing a molded body. The molding material according to the present disclosure can also be said to be a material obtained by chemically modifying a polypeptide. The shape of the molded body in the present specification is not particularly limited, and may be, for example, a film shape, a plate shape, a block shape, a sponge shape, or a fiber shape. The form of the molding material in the present specification is not limited at all, and may be, for example, a powder, a particle, a liquid, or a gel. The molding material according to the present disclosure can be formed into molded bodies having various shapes by, for example, heat-and-pressure molding, cast molding, or spinning. At the time of molding, a mold may be used as necessary.

The polymer compound production method according to the present disclosure utilizes a reaction between a mercapto group (nucleophilic functional group) of a polypeptide and a skeleton of a polyether or the like having a predetermined unsaturated bond (electrophilic functional group).

A first embodiment of the polymer compound production method includes subjecting a polypeptide having at least one mercapto group (hereinafter, also referred to as component (A)) and at least one compound selected from the group consisting of a polyether, a polyester, and a polycarbonate and having two structures represented by the following formula (1) (hereinafter, also referred to as component (B1), also simply referred to as component (B) together with component (B2) described later) to a heating reaction in dimethyl sulfoxide in the presence of a base (hereinafter, also referred to as component (C)) and a reducing agent (hereinafter, also referred to as component (D)). The heating reaction is a reaction between component (A) and component (B), and is performed, for example, by performing heating to 50°C or higher. Note that a heating timing is not limited to a timing after components (A) to (D) are mixed, and for example, components (A) to (D) may be added using dimethyl sulfoxide heated in advance, heating may be started after components (C) and (D) are added to dimethyl sulfoxide and components (A) and (B) may be added and caused to react after a predetermined temperature is reached, or heating may be started after one of components (A) and (B) and components (C) and (D) are added, and the remaining one of components (A) and (B) may be added and caused to react after a predetermined temperature is reached. In a case of production conditions in which other conditions are common, a desired effect of the present disclosure can be obtained at a higher level by blending component (C), but component (C) can be omitted depending on a level of the desired effect.

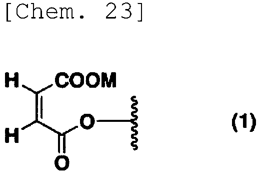

The number of mercapto groups of the polypeptide (component (A)) is 1 or more per molecule, but may be, for example, 2 or more, 4 or more, 8 or more, or 16 or more. When the number of mercapto groups is within the above range, a contact probability of a reaction point is improved, and the reaction can be allowed to proceed efficiently. The number of mercapto groups of component (A) may be, for example, 64 or less or 32 or less per molecule. When the number of mercapto groups is within the above range, it is possible to more efficiently obtain a target polymer compound while suppressing generation of a byproduct formed by a reaction between mercapto groups of the polypeptide.

The number of amino acid residues constituting component (A) may be, for example, 50 or more, 100 or more, 150 or more, 200 or more, 250 or more, 300 or more, 350 or more, 400 or more, 450 or more, or 500 or more. The number of amino acid residues may be, for example, 5000 or less, 4500 or less, 4000 or less, 3500 or less, 3000 or less, 2500 or less, 2000 or less, 1500 or less, or 1000 or less. As the number of amino acid residues is smaller, solubility in dimethyl sulfoxide tends to increase. In a case where the number of amino acid residues constituting component (A) is, for example, 5000 or less or 2500 or less, when the polypeptide is dissolved in a solvent to cause the polypeptide to react with the compound (component (B1)), production efficiency of a target polymer compound can be further improved. In addition, a molded body containing the resulting polymer compound can exhibit mechanical strength and flexibility at a higher level.

Component (A) may be, for example, a hydrophobic polypeptide. When component (A) is a hydrophobic polypeptide, preparation of component (A) is easier, and therefore raw material cost in the above-described production method can be further reduced. In this case, a molded body containing the resulting polymer compound can exhibit better flexibility. The molded body can also exhibit better water resistance. In this case, the resulting molded body can have a longer service life. Note that hydrophobicity of the polymer compound can be arbitrarily adjusted by preparing hydrophobicity of component (A).

The hydrophobicity of component (A) can be estimated using a value of an average hydropathy index (hereinafter, also referred to as "HI") described later as an index. A value of average HI of component (A) may be more than 0, and may be, for example, 0.10 or more, 0.20 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. An upper limit value of average HI of component (A) is not particularly limited, and may be, for example, 1.00 or less or 0.7 or less.

Component (A) preferably has low solubility in a lithium bromide aqueous solution (concentration: 9 M) at 60°C. This solubility can be evaluated using a polypeptide corresponding to component (A). In a case where the polypeptide is dissolved in a lithium bromide aqueous solution (concentration: 9 M) at 60°C, a maximum concentration may be, for example, less than 30% by mass, less than 25% by mass, less than 20% by mass, less than 15% by mass, less than 10% by mass, less than 5% by mass, or less than 1% by mass. Note that component (A) may be completely insoluble in a lithium bromide aqueous solution (concentration: 9 M) at 60°C. For a polymer compound obtained by the production method according to the present disclosure, the solubility of a polypeptide moiety of the polymer compound can be confirmed by using a polypeptide obtained by decomposing the polymer compound and isolating only a hydrophobic polypeptide.

Component (A) preferably has a large water contact angle. The water contact angle can be evaluated using a polypeptide corresponding to component (A) on a base material. More specifically, a membrane containing the above polypeptide is formed and can be evaluated using the membrane. Component (A) is preferably a polypeptide constituting a membrane having a contact angle of 55° or more five seconds after dropping water onto the membrane. The contact angle may be, for example, 60° or more, 65° or more, or 70° or more. For a polymer compound obtained by the production method according to the present disclosure, the contact angle of a polypeptide moiety of the polymer compound can be confirmed by using a polypeptide obtained by decomposing the polymer compound and isolating only a hydrophobic polypeptide.

Component (A) preferably has excellent hot water resistance. The hot water resistance can be evaluated using a polypeptide corresponding to component (A). Component (A) is preferably a polypeptide that is not decomposed even when a dispersion containing the polypeptide and water, in which a content of the polypeptide is 5% by mass, is prepared, and the dispersion is heated at 100°C for five hours. For a polymer compound obtained by the production method according to the present disclosure, the hot water resistance of a polypeptide moiety of the polymer compound can be confirmed by using a polypeptide obtained by decomposing the polymer compound and isolating only a hydrophobic polypeptide.

A weight average molecular weight of component (A) is, for example, 200 to 1,000,000, more preferably 300 to 900,000, still more preferably 400 to 800,000, still more preferably 500 to 700,000, still more preferably 600 to 600,000, still more preferably 1,000 to 600,000, still more preferably 3,000 to 600,000, still more preferably 5,000 to 600,000, still more preferably 10,000 to 600,000, and still more preferably 5,000 to 100,000. Note that when the weight average molecular weight of component (A) is less than 200, the polypeptide functioning as a hard segment may be too small with respect to a polyether structure or the like (soft segment) of component (B1). In this case, rigidity of a molded body molded using the resulting polymer compound (molding material) is small, and there is a possibility that it is difficult to use the molded body, for example, as a structural body.

The weight average molecular weight in the present specification is a value measured by sodium dodecyl sulfate-polyacrylamide gel electrophoresis (SDS-PAGE). Note that the electrophoresis is performed in the following procedure. That is, first, 200 µL of 2 M lithium chloride DMSO (manufactured by FUJIFILM Wako Pure Chemical Corporation) is added to 2 mg of a powder sample, and the mixture is stirred while being heated at 80°C for 60 minutes and further at 95°C for ten minutes to dissolve the sample. Thereafter, the sample is diluted to 50 times with a 10 M urea solution, further diluted to 2 times with a sample buffer (manufactured by FUJIFILM Wako Pure Chemical Corporation), and heated at 95°C for 5 minutes to denature the protein. Subsequently, a gel for SDS-PAGE (manufactured by Bio-lad) is attached to an electrophoresis device (manufactured by Bio-lad), the device is filled with an SDS buffer, and the electrophoresis device is connected to a power supply device (manufactured by Bio craft). 10 µL of the denatured sample is added to each well of the gel for SDS-PAGE, and a current of 30 mA/sheet is allowed to flow for 30 minutes. After completion of the electrophoresis, the gel for SDS-PAGE is taken out from the device, immersed in Oriole fluorescent gel stain (manufactured by Bio-lad), and shaken for one hour. Thereafter, the gel is placed on a UV sample tray (manufactured by Bio-lad), and a stained image is acquired with a Gel Doc EZ gel imaging device (manufactured by Bio-lad). Note that XL ladder Broad (manufactured by Apo Science) was used as a standard sample.

A molecular weight of component (A) may be, for example, 2 kDa or more, 3 kDa or more, 4 kDa or more, 5 kDa or more, 6 kDa or more, 7 kDa or more, 8 kDa or more, 9 kDa or more, 10 kDa or more, 20 kDa or more, 30 kDa or more, 40 kDa or more, 50 kDa or more, 60 kDa or more, 70 kDa or more, 80 kDa or more, 90 kDa or more, or 100 kDa or more. The molecular weight of component (A) may be, for example, 500 kDa or less, 400 kDa or less, less than 360 kDa, 300 kDa or less, or 200 kDa or less. The molecular weight of component (A) may be adjusted within the above-described range, and may be, for example, 2 to 500 kDa. Note that the molecular weight can be measured by a liquid chromatograph mass spectrometer.

Component (A) may be a natural protein or an artificial protein. In addition, component (A) may be a protein (modified protein) obtained by chemically modifying (for example, alkylating, etherifying, esterifying, amidating, thioetherifying, thioesterifying, and the like) a natural protein or an artificial protein. Amino acid sequences of the natural protein, the artificial protein, and the modified protein are not particularly limited as long as each of the natural protein, the artificial protein, and the modified protein has one or more mercapto groups.

As component (A), for example, a protein having close physical properties can be adopted according to required properties suitable for an application of a polymer compound. Examples of the polypeptide according to the present embodiment include a polypeptide that can be used for medical use and a polypeptide that can be used for industrial use. The phrase "can be used for industrial use" means, for example, that it can be used for various general-purpose materials used indoors or outdoors.

Specific examples of the polypeptide that can be used for medical use include an enzyme, a regulatory protein, a receptor, a peptide hormone, a cytokine, a membrane or a transport protein, an antigen used for vaccination, a vaccine, an antigen-binding protein, an immunostimulatory protein, an allergen, and a full length antibody or an antibody fragment or a derivative thereof.

Specific examples of the polypeptide that can be used for industrial use include a structural protein. The structural protein means a protein related to a structure of a living body, a protein constituting a structure created by a living body, or a protein derived from these proteins. The structural protein also refers to a protein that self-aggregates under predetermined conditions to form a structure such as a fiber, a film, a resin, a gel, a micelle, or a nanoparticle. Furthermore, the structural protein can also be said to be a protein in which a motif formed of a characteristic amino acid sequence or an amino acid number residue is repeatedly present to form a skeleton of an organism or a material. Specific examples of the structural protein include spider silk, silkworm silk, keratin, collagen, elastin, resilin, and a protein derived from these. The structural protein may be an artificial fibroin or an artificial spider silk fibroin (artificial modified spider silk fibroin).

The structural protein may be an artificial structural protein. The artificial structural protein includes a synthetic protein and a recombinant structural protein produced from a microorganism by genetic recombination technology. That is, in the present specification, the "artificial structural protein" means a structural protein artificially produced. The artificial structural protein may have the same amino acid sequence as that of a natural structural protein, or may be a modified structural protein in which a part of an amino acid sequence of a naturally derived structural protein is modified depending on the amino acid sequence from a viewpoint of productivity, moldability, or the like.

A glycine residue content of the artificial structural protein may be 10 to 55%. The glycine residue content may be, for example, 13% to 55%, 15% to 55%, 18% to 55%, 20% to 55%, 22% to 55%, or 25% to 55%. Note that, in the present specification, the "glycine residue content" is a value represented by the following formula.
Glycine residue content = (Number of glycine residues contained in artificial structural protein/Number of all amino acid residues of polypeptide) × 100 (%)

The artificial structural protein may have 150 or more amino acid residues. The number of amino acid residues may be, for example, 200 or more or 250 or more and is preferably 300 or more, 350 or more, 400 or more, 450 or more, or 500 or more.

In the artificial structural protein, the total content of the content of at least one type of amino acid residue selected from the group consisting of serine, threonine, and tyrosine (that is, any one of a serine residue content, a threonine residue content, a tyrosine residue content, the sum of the serine residue content and the threonine residue content, the sum of the serine residue content and the tyrosine residue content, the sum of the threonine residue content and the tyrosine residue content, and the sum of the serine residue content, the threonine residue content, and the tyrosine residue content), an alanine residue content, and a glycine residue content may be 40% or more. The total content may be, for example, 45% or more, 50% or more, 55% or more, or 60% or more. An upper limit of the total content is not particularly limited, but may be, for example, 90% or less, 85% or less, or 80% or less.

In the artificial structural protein, the sum of the serine residue content, the threonine residue content, and the tyrosine residue content may be 4% or more, 4.5% or more, 5% or more, 5.5% or more, 6% or more, 6.5% or more, or 7% or more. The sum of the serine residue content, the threonine residue content, and the tyrosine residue content may be, for example, 35% or less, 33% or less, 30% or less, 25% or less, or 20% or less.

The artificial structural protein has an average distribution of serine residues, threonine residues, or tyrosine residues, and the total content of the serine residue, the threonine residue, and the tyrosine residue in any consecutive 20 amino acid residues may be 4% or more, 5% or more, 10% or more, or 15% or more, and may be 50% or less, 40% or less, 30% or less, or 20% or less.

In addition, the alanine reside content, the serine residue content, the threonine residue content, and the tyrosine residue content have the same meaning as those obtained by replacing the alanine residue with a glycine residue, a serine residue, a threonine residue, and a tyrosine residue, respectively, in the above formula.

The artificial structural protein may have a repetitive sequence. That is, the artificial structural protein may have a plurality of amino acid sequences (repetitive sequence units) having a high sequence identity in the artificial structural protein. The number of amino acid residues of the repetitive sequence unit is preferably 6 to 200.

The total number of glycine residues, serine residues, glutamine residues and alanine residues to the total number of amino acid residues in the repetitive sequence unit may be 40% or more, 45% or more, 50% or more, 55% or more, 60% or more, 65% or more, or 70% or more. Sequence identity between the repetitive sequence units may be, for example, 80% or more, 85% or more, 90% or more, 95% or more, 96% or more, 97% or more, 98% or more, or 99% or more.

A hydropathy index of the repetitive sequence unit may be, for example, -0.80 or more, -0.70 or more, -0.60 or more, -0.50 or more, -0.40 or more, -0.30 or more, -0.20 or more, -0.10 or more, 0.00 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. Note that an upper limit value of the hydropathy index of the repetitive sequence unit is not particularly limited, and may be, for example, 1.0 or less or 0.7 or less.

The artificial structural protein may contain an (A)n motif. In the present specification, the (A)n motif means an amino acid sequence mainly containing an alanine residue. The number of amino acid residues in the (A)n motif may be 2 to 27 and may be an integer of 2 to 20, 2 to 16, or 2 to 12.

In addition, a ratio of the number of alanine residues to the total number of amino acid residues in the (A)n motif only needs to be 40% or more, and may be 45% or more, 50% or more, 55% or more, 60% or more, 65% or more, 70% or more, 80% or more, 83% or more, 85% or more, 86% or more, 90% or more, 95% or more, or 100% (which means that the (A)n motif consists only of alanine residues).

In the (A)n motif, the total number of alanine residues, serine residues, threonine residues, and valine residues to the total number of amino acid residues in the (A)n motif may be 80% or more, but is preferably 85% or more, more preferably 90% or more, still more preferably 95% or more, and further still more preferably 100% (which means that the (A)n motif consists only of one or more amino acid residues selected from an alanine residue, a serine residue, a threonine residue, and a valine residue). A plurality of (A)n motifs present in the recombinant structural protein according to the present embodiment may have the same amino acid sequence or different amino acid sequences. Note that the (A)n motif mainly contains alanine residues and therefore tends to have an α-helix structure or a β-sheet structure. By inclusion of the (A)n motif in the repetitive sequence unit, the artificial structural protein according to the present embodiment has these repetitive secondary structures. Therefore, as described later, when the artificial structural protein is in a form of a molded body such as a fiber, a film, or a resin, the artificial structural protein is expected to exhibit high strength due to these secondary structures.

Note that when the structural protein is molded, an amino acid having a relatively smaller side chain is more likely to form a hydrogen bond, and a molded body having higher strength is more likely to be obtained. In addition, an alanine residue and a glycine residue are amino acids having nonpolar side chains, and therefore are arranged so as to face inward in a folding process during polypeptide production and easily have an α-helix structure or a β-sheet structure. Therefore, a ratio of an amino acid such as a glycine residue or an alanine residue is desirably high. An alanine residue content only needs to be, for example, 10 to 40%, and may be 12 to 40%, 15 to 40%, 18 to 40%, 20 to 40%, or 22 to 40% from a viewpoint of obtaining a molded body having better strength. A glycine residue content only needs to be, for example, 10 to 55%, and may be 11 to 55%, 13 to 55%, 15 to 55%, 18 to 55%, 20 to 55%, 22 to 55%, or 25 to 55% from a viewpoint of obtaining a molded body having better strength.

In the present specification, the "alanine residue content" means the number of alanine residues to the total number of amino acid residues constituting a protein, and is a value represented by the following formula.
Alanine residue content = (number of alanine residues contained in protein/total number of amino acid residues of protein) × 100 (%)

In addition, a glycine residue content, a serine residue content, a threonine residue content, a proline residue content, and a tyrosine residue content have the same meanings as those obtained by replacing the alanine residue with the glycine residue, the serine residue, the threonine residue, the proline residue, and the tyrosine residue, respectively, in the above formula.

In the structural protein, an amino acid having a relatively large side chain or an amino acid having flexibility is preferably homogenously contained in the entire sequence to a certain extent. Specifically, the structural protein may include a motif containing a tyrosine residue, a threonine residue, or a proline residue repeatedly in a cycle. When such a structural protein is used, formation of strong intermolecular hydrogen bonding is easily inhibited during processing of a molded body obtained by molding, and processability is easily improved. For example, the sum of a proline residue content, a threonine residue content, and a tyrosine residue content in any consecutive 20 amino acid residues may be 5% or more, more than 5.5%, 6.0% or more, more than 6.5%, 7.0% or more, more than 7.5%, 8.0% or more, more than 8.5%, 9.0% or more, 10.0% or more, or 15.0% or more. In addition, for example, the sum of a proline residue content, a threonine residue content, and a tyrosine residue content in any consecutive 20 amino acid residues may be 50% or less, 40% or less, 30% or less, or 20% or less.

In the artificial structural protein, similarly to the protein described above, the total content of a serine residue, a threonine residue, and a tyrosine residue may be, for example, 4% or more, 4.5% or more, 5% or more, 5.5% or more, 6% or more, 6.5% or more, or 7% or more from a viewpoint of improving productivity of a reaction-modified protein or the like. The total content of a serine residue, a threonine residue, and a tyrosine residue may be, for example, 35% or less, 33% or less, 30% or less, 25% or less, or 20% or less.

In the production method, the blending amount of the polypeptide (component (A)) may be, for example, more than 1 part by mass, 2 parts by mass or more, 5 parts by mass or more, more than 5 parts by mass, 6 parts by mass or more, 7 parts by mass or more, or 8 parts by mass or more with respect to 100 parts by mass of dimethyl sulfoxide. With the production method according to the present disclosure, it is possible to suppress formation of a disulfide bond between polypeptides, and thus it is possible to promote production of a polymer compound in a situation where the concentration of the polypeptide is increased, and it is possible to perform production with higher efficiency. The blending amount of component (A) may be, for example, 50 parts by mass or less, 30 parts by mass or less, 20 parts by mass or less, or 15 parts by mass or less with respect to 100 parts by mass of dimethyl sulfoxide. When an upper limit value of the blending amount of component (A) is within the above range, the reaction can be allowed to proceed while more sufficiently suppressing generation and gelation of a by-product. In the production method, a ratio of the polypeptide (component (A)) in the dimethyl sulfoxide solution may be, for example, 15% by mass or less, less than 15% by mass, 14% by mass or less, 12.5% by mass or less, or 11.5% by mass or less.

At least one compound (component (B1)) selected from the group consisting of a polyether, a polyester, and a polycarbonate and having two structures represented by formula (1) is a compound capable of reacting with a mercapto group of the above-described polypeptide and capable of introducing a polyether structure, a polyester structure, or a polycarbonate structure into the polypeptide by having the structure represented by formula (1).

Examples of the polyether having two structures represented by formula (1) include polyethylene glycol, polytetramethylene glycol, polypropylene glycol, and an ethylene glycol-propylene glycol copolymer. Examples of the polyester having two structures represented by formula (1) include polylactic acid, polyglycolic acid, polybutylene succinate, polycaprolactone, and polyhydroxyalkanoates including a 3-hydroxybutanoic acid-3-hydroxyhexanoic acid copolymer and the like. Examples of the polycarbonate having two structures represented by formula (1) include aliphatic polycarbonates such as polyethylene carbonate, polypropylene carbonate, and polytrimethylene carbonate. Since these compounds are compounds that are easier in molecular motion than the above-described polypeptide, when each of these compounds is a compound as described above, flexibility of a molded body containing the resulting polymer compound can be further improved.

In the production method, a weight average molecular weight of the compound (component (B1)) may be, for example, 100 or more, 1000 or more, or 2000 or more. By using a compound having a weight average molecular weight within the above range as component (B1), an influence of bulkiness between molecules can be further suppressed, and the reaction can be allowed to proceed efficiently. In the above production method, the weight average molecular weight of component (B1) may be, for example, 200,000 or less, 100,000 or less, 50,000 or less, or 10,000 or less. By using a compound having a weight average molecular weight within the above range as component (B1), a decrease in solubility of the compound in a solvent and a decrease in reactivity with the polypeptide can be more sufficiently suppressed.

In the production method, the weight average molecular weight of the compound (component (B1)) may be, for example, 0.005 times or more, 0.01 times or more, 0.05 times or more, or 0.1 times or more based on a weight average molecular weight of the polypeptide. By setting a relationship in weight average molecular weight between the polypeptide and component (B1) within the above range, it is possible to produce a synthetic polymer having a polyether structure or the like having a sufficient length with respect to a polypeptide moiety. A molded body containing such a synthetic polymer can have excellent flexibility. In the production method, the weight average molecular weight of component (B1) may be, for example, 20 times or less, 10 times or less, 5 times or less, or 2 times or less based on the weight average molecular weight of the polypeptide. By setting the relationship in weight average molecular weight between the polypeptide and component (B) within the above range, the reaction can be allowed to proceed while further suppressing a decrease in reactivity. In the production method, the weight average molecular weight of component (B1) may be adjusted within the above-described range, and may be, for example, 0.005 to 20 times, 0.01 to 10 times, 0.05 to 5 times, or 0.1 to 2 times based on the weight average molecular weight of the polypeptide.

In the production method, a ratio of the number of moles of the structure represented by formula (1) and included in the compound (component (B1)) to the number of moles of mercapto groups of the polypeptide (component (A)) may be, for example, 0.1 times or more, 0.2 times or more, 0.5 times or more, or 0.7 times or more. By setting the ratio within the above range, the reaction can be allowed to proceed while further suppressing a decrease in reactivity. In the production method, a ratio of the number of moles of the structure represented by formula (1) and included in component (B1) to the number of moles of mercapto groups of component (A) may be, for example, 5.0 times or less, 3.0 times or less, 1.5 times or less, or 0.8 times or less. By setting the ratio within the above range, the reaction can be allowed to proceed while further suppressing generation and gelation of a by-product. By adjusting the molar ratio, the number of polyether structures and the like in the resulting polymer compound can be prepared. In the resulting polymer compound, the number of polyether structures and the like with respect to one polypeptide can be, in total, for example, 1 or more or 2 or more, and can be 2 to 10, 2 to 8, 2 to 6, or 2 to 4.

The number of moles of mercapto groups in the present specification means a value measured by cutting out a polypeptide in a measurement sample by an enzyme, converting the polypeptide into an amino acid, and then separating and detecting the amino acid by a liquid chromatograph. Note that when the polypeptide is prepared by oneself, the number of moles of the functional group can be determined from a sequence of a designed molecule.

The number of moles of each of the structure represented by general formula (1) and the structure represented by general formula (2) in the present specification means a value measured by MALDI-TOFMS.

The number of moles of the compound (component (B1)) present in dimethyl sulfoxide in the production method may be, for example, 0.1 times or more, 0.2 times or more, 0.5 times or more, or 0.8 times or more the total number of moles of mercapto groups of the polypeptide. By adjusting the raw material blending ratio such that the number of moles of component (B1) is large with respect to the total number of moles of mercapto groups, a reaction probability between the polypeptide and the compound can be improved, and a frequency of formation of a disulfide bond between the polypeptides can be more sufficiently reduced. The number of moles of component (B1) present in dimethyl sulfoxide in the production method may be, for example, 5.0 times or less, 3.0 times or less, 1.5 times or less, or 1.0 times or less the total number of moles of mercapto groups of the polypeptide. By setting the number of moles of component (B1) within the above range, the reaction can be allowed to proceed while further suppressing generation and gelation of a by-product.

The base (component (C)) in the above-described production method is a compound capable of activating a mercapto group of the polypeptide to promote the Michael addition reaction with the functional group represented by formula (1). Examples of the base include a primary amine, a secondary amine, a tertiary amine, a nitrogen-containing cyclic compound, a nitrogen-containing aromatic compound, and an inorganic base. Examples of the primary amine include ethanolamine and hexamethylenediamine. Examples of the secondary amine include diethylamine, methylethylamine, and N-methylbutylamine. Examples of the tertiary amine include triethylamine, diethylmethylamine, and (N,N-diisopropylethylamine (DIPEA). Examples of the nitrogen-containing cyclic compound include quinuclidine, 1,4-diazabicyclo [2.2.2] octane (DABCO), 1,8-diazabicyclo [5.4.0] undec-7-ene (DBU), and 1,5-diazabicyclo [4.3.0] non-5-ene (DBN). Examples of the nitrogen-containing aromatic compound include pyridine and imidazole. Examples of the inorganic base include sodium hydroxide, potassium hydroxide, sodium hydrogen carbonate, potassium hydrogen carbonate, sodium carbonate, potassium carbonate, cesium carbonate, tripotassium phosphate, sodium acetate, and potassium acetate.

A lower limit value of the blending amount of the base may be, for example, 0.01 parts by mass or more, 0.05 parts by mass or more, 0.1 parts by mass or more, or 0.5 parts by mass or more based on the mass of the polypeptide. By setting the lower limit value of the blending amount of the base within the above range, the reaction between a mercapto group of the polypeptide and the unsaturated bond represented by formula (1) or (2) included in the compound can be further promoted. An upper limit value of the blending amount of the base may be, for example, 50 parts by mass or less, 20 parts by mass or less, 10 parts by mass or less, or 5 parts by mass or less based on the mass of the polypeptide. By setting the upper limit value of the blending amount of the base within the above range, decomposition of a molecule due to an influence of the remaining base can be further suppressed, and a target polymer compound can be synthesized. The blending amount of the base may be adjusted within the above-described range, and may be, for example, 0.05 to 50 parts by mass or 0.1 to 10 parts by mass based on the mass of the polypeptide. The blending amount of the base may be, for example, 0 to 50 parts by mass, 0 to 10 parts by mass, 0 to 1 parts by mass, or 0 to 0.5 parts by mass based on the mass of the polypeptide.

The reducing agent (component (D)) in the above-described production method is a compound capable of stabilizing a mercapto group of the polypeptide in dimethyl sulfoxide and inhibiting formation of a disulfide bond between the mercapto groups. The reducing agent may contain, for example, at least one selected from the group consisting of a thiol, a dithiol, sodium sulfite, sodium hyposulfite, sodium sulfate, and sodium dithionite, and the reducing agent may contain at least one selected from the group consisting of a dithiol and sodium sulfite, and may be a dithiol or sodium sulfite. The reducing agent is preferably a compound having a mercapto group. The reducing agent is preferably a dithiol.

The compound having a mercapto group is desirably a compound represented by the following general formula (Y). By using such a compound, a chemically stable six-membered ring structure is formed in an oxidized form after the reduction reaction, occurrence of a reaction between the reducing agent and a Michael addition receptor is suppressed in the reaction system, for example, by forming a cyclic structure between the reducing agent and a mercapto group of the polypeptide, formation of a disulfide bond between the polypeptides is suppressed, the reaction between a mercapto group of the polypeptide and the Michael addition receptor is significant, and a target polymer compound according to reaction design can be more efficiently produced. Examples of the dithiol include dithiothreitol and 1,4-butanedithiol. The compound (dithiol) represented by general formula (Y) is specifically dithiothreitol or 1,4-butanedithiol.

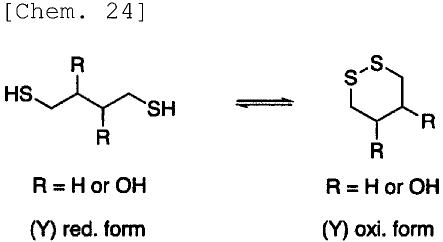

Examples of other compounds having a mercapto group include 3-mercaptopropionic acid, 3-mercapto-1,2-propanediol, and pentaerythritol tetra(3-mercaptopropionate). The reducing agent may be, for example, (tris(2-carboxyethyl) phosphine (TCEP) in addition to the above compounds.

A lower limit value of the blending amount of the reducing agent may be, for example, 0.1 equivalents or more, 0.3 equivalents or more, 0.5 equivalents or more, 0.8 equivalents or more, or 1.0 equivalents or more with respect to a mercapto group of the polypeptide. By setting the lower limit value of the blending amount of the reducing agent within the above range, formation of a disulfide bond by a reaction between mercapto groups of the polypeptide can be more sufficiently inhibited. This can further improve production efficiency of a target synthetic polymer. An upper limit value of the blending amount of the reducing agent may be, for example, 2.4 equivalents or less, 2.0 equivalents or less, 1.6 equivalents or less, 1.2 equivalents or less, or 1.1 equivalents or less with respect to a mercapto group of the polypeptide. By setting the upper limit value of the blending amount of the reducing agent within the above range, the reaction can be allowed to proceed while further suppressing the reaction between component (B1) and the reducing agent and generation of a side reactant.

In the production method, the reaction between the polypeptide (component (A)) and the compound (component (B1)) may be performed, for example, at 55°C or higher. In a situation where the reaction temperature exceeds 55°C, formation of a disulfide bond between mercapto groups of the polypeptide can usually occur in dimethyl sulfoxide. On the other hand, in the production method according to the present disclosure, the formation of the disulfide bond can be eliminated by performing the reaction in the presence of a reducing agent. Therefore, activity of the reaction between the polypeptide and the compound can be improved in a state where the formation of the disulfide bond is suppressed, and efficiency of synthesis of a polymer compound can be improved.

In the above production method, the reaction temperature of the reaction between component (A)) and component (B1) may be, for example, 20°C or higher, 30°C or higher, 40°C or higher, 50°C or higher, 53°C or higher, 55°C or higher, 57°C or higher, or 60°C or higher. When the lower limit value of the reaction temperature is within the above range, the reaction between component (A) and component (B1) can be further promoted. In the above production method, an upper limit value of the reaction temperature of the reaction between component (A)) and component (B1) may be, for example, 110°C or lower, 100°C or lower, 90°C or lower, 85°C or lower, 80°C or lower, 75°C or lower, or 70°C or lower. When the upper limit value of the reaction temperature is within the above range, formation of a bond between mercapto groups of the polypeptide as a side reaction can be further suppressed, and a target polymer compound can be efficiently synthesized. The reaction temperature of the reaction between component (A)) and component (B1) may be adjusted within the above-described range, and may be, for example, 50 to 90°C, 53 to 80°C, 55 to 75°C, or 55 to 70°C. The reaction proceeds at any temperature, but a temperature at which a DMSO solvent is not frozen and a protein is not decomposed is desirable.

The polymer compound production method may further include another step. Examples of the other step include separately preparing component (A) and separately preparing component (B1).

A method for preparing component (A) may be a method including causing a host transformed with an expression vector described later to express a nucleic acid. At this time, component (A) may be an artificial fibroin. Examples of an expression method include not only direct expression but also secretory production and fusion protein expression according to a method described in Molecular Cloning, 2nd edition or the like. When expression is performed by yeast, an animal cell, or an insect cell, component (A) can be obtained as a polypeptide to which a sugar or a sugar chain is added.

The artificial fibroin can be produced by, for example, culturing a host transformed with an expression vector in a culture medium, producing and accumulating the artificial fibroin in the culture medium, and collecting the artificial fibroin from the culture medium. A method for culturing the host in the culture medium can be performed according to a method usually used for culturing a host.

When the host is a prokaryote such as Escherichia coli or a eukaryote such as yeast, as the culture medium of the host, either a natural medium or a synthetic medium may be used as long as the medium contains a carbon source, a nitrogen source, an inorganic salt, and the like that can be assimilated by the host, and can efficiently culture the host.

As the carbon source, any carbon source that can be assimilated by the host can be used. Examples of the carbon source include a carbohydrate such as glucose, fructose, sucrose, molasses containing glucose, fructose, and sucrose, starch, or starch hydrolysate, an organic acid such as acetic acid or propionic acid, and alcohols such as ethanol and propanol.

Examples of the nitrogen source include an ammonium salt of an inorganic or organic acid, such as ammonia, ammonium chloride, ammonium sulfate, ammonium acetate, or ammonium phosphate and other nitrogen-containing compounds, peptone, meat extract, yeast extract, corn steep liquor, casein hydrolysate, soybean cake, soybean cake hydrolysate, and various fermenters and digests thereof.

Examples of the inorganic salt include potassium dihydrogen phosphate, dipotassium phosphate, magnesium phosphate, magnesium sulfate, sodium chloride, ferrous sulfate, manganese sulfate, copper sulfate, and calcium carbonate.

Prokaryotes such as Escherichia coli or eukaryotes such as yeast can be cultured, for example, under aerobic conditions such as shaking culture or aeration agitation submerged culture. A culture temperature is, for example, 15 to 40°C. A culture time is usually 16 hours to seven days. The pH of a culture medium during culture is preferably maintained at 3.0 to 9.0. The pH of the culture medium is adjusted using, for example, an inorganic acid, an organic acid, an alkali solution, urea, calcium carbonate, and ammonia.

In addition, an antibiotic such as ampicillin or tetracycline may be added to the culture medium as necessary during culture. When a microorganism transformed with an expression vector using an inducible promoter as a promoter is cultured, an inducer may be added to the medium as necessary. For example, when a microorganism transformed with an expression vector using a lac promoter is cultured, isopropyl-β-D-thiogalactopyranoside or the like may be added to the medium, and when a microorganism transformed with an expression vector using a trp promoter is cultured, indole acrylic acid or the like may be added to the medium.

Examples of a culture medium for insect cells include a commonly used TNM-FH medium (manufactured by Pharmingen Inc.), an Sf-900 II SFM medium (manufactured by Life Technologies Corporation), ExCell 400 and ExCell 405 (both manufactured by JRH Biosciences Inc.), and Grace's Insect Medium (Nature, 195, 788 (1962)).

The insect cells can be cultured for a culture time of one to five days, for example, under conditions such as a pH of a culture medium of 6 to 7 and a culture temperature of 25 to 30°C. In addition, an antibiotic such as gentamicin may be added to the culture medium as necessary during culture.

When the host is a plant cell, the transformed plant cell may be cultured as it is, or can be differentiated into an organ of a plant and cultured. Examples of the medium for culturing a plant cell include commonly used a Murashige and Skoog (MS) medium, a White medium, and a medium in which a plant hormone such as auxin or cytokinin is added to these media.

The animal cells can be cultured for a culture time of three to 60 days, for example, under conditions such as a pH of a culture medium of 5 to 9 and a culture temperature of 20 to 40°C. In addition, an antibiotic such as kanamycin or hygromycin may be added to the medium as necessary during culture.

Examples of a method for producing an artificial fibroin using a host transformed with the expression vector include a method for producing the artificial fibroin in a host cell, a method for secreting the artificial fibroin outside the host cell, and a method for producing the artificial fibroin on an outer membrane of the host cell. Each of these methods can be selected depending on a host cell to be used and a structure of an artificial fibroin to be produced.

For example, when an artificial fibroin is produced in a host cell or on an outer membrane of the host cell, the production method can be altered to actively secrete the artificial fibroin outside the host cell according to the method of Paulson et al. (J. Biol. Chem., 264, 17619 (1989)), the method of Lowe et al. (Proc. Natl. Acad. Sci. USA, 86, 8227 (1989), Genes Develop., 4, 1288 (1990)), or the methods described in JP 05-336963 A, WO 1994/023021 A, and the like. That is, the artificial fibroin can be actively secreted outside the host cell by expressing the artificial fibroin in a form in which a signal peptide is added to a polypeptide containing an active site of the artificial fibroin using a gene recombination technique.

The artificial fibroin produced by a host transformed with the expression vector can be isolated and purified by a method usually used for protein isolation and purification. For example, when the artificial fibroin is expressed in a state of being dissolved in a cell, after completion of culture, the host cell is collected by centrifugation and suspended in an aqueous buffer, and then, disrupted with an ultrasonicator, a French press, a Manton-Gaulin homogenizer, a Dyno-Mill, or the like, thereby obtaining a cell-free extract. A purified preparation is obtained from supernatant obtained by centrifuging the cell-free extract according to one of the following methods usually used for protein isolation and purification or according to a combination of the following methods, that is, solvent extraction, salting-out using ammonium sulfate or the like, desalting, precipitation using an organic solvent, anion exchange chromatography using a resin such as diethylaminoethyl (DEAE)-sepharose or DIAION HPA-75 (manufactured by Mitsubishi Kasei Kogyo Kabushiki Kaisha), cation exchange chromatography using a resin such as S-sepharose FF (manufactured by Pharmacia Corporation), hydrophobic chromatography using a resin such as butyl sepharose or phenyl sepharose, gel filtration using a molecular sieve, affinity chromatography, chromatofocusing, and electrophoresis such as isoelectric focusing phoresis.

As the chromatography, column chromatography using phenyl-TOYOPEARL (Tosoh Corporation), DEAE-TOYOPEARL (Tosoh Corporation), and Sephadex G-150 (Pharmacia Biotech Inc.) is preferably used.

When the artificial fibroin is expressed by formation of an insoluble matter in a cell, similarly, the host cells are collected, disrupted and centrifuged to collect the insoluble matter of the artificial fibroin as a precipitated fraction. The collected insoluble matter of the artificial fibroin can be solubilized with a protein denaturing agent. After this operation, a purified preparation of the artificial fibroin can be obtained by an isolation and purification method similar to that described above.

When the artificial fibroin or a derivative in which a sugar chain is added to the artificial fibroin is secreted extracellularly, the artificial fibroin or the derivative thereof can be collected from culture supernatant. That is, culture supernatant is acquired by treating a culture by a method such as centrifugation, and a purified preparation can be obtained from the culture supernatant using an isolation and purification method similar to that described above.

Component (A) may be an artificial fibroin. When the artificial fibroin is separately prepared, a method therefor may be, for example, a method for artificially synthesizing a naturally derived fibroin. Here, examples of the naturally derived fibroin include a fibroin produced by an insect or a spider. A natural fibroin is a fibrous protein, has a molecular weight of about 370,000, is constituted by two subunits, and has a high content of a glycine residue, an alanine residue, a serine residue, and a tyrosine residue, and these amino acid residues account for nearly 90% of the total number of amino acid residues. The natural fibroin has a crystalline region rich in amino acid residues having relatively small side chains, such as glycine, alanine, and serine, and an amorphous region having amino acid residues having relatively large side chains, such as tyrosine.

More specific examples of the naturally derived fibroin include a fibroin whose sequence information is registered in NCBI GenBank. For example, it can be confirmed by extracting a sequence in which spidroin, ampullate, fibroin, "silk and polypeptide", or "silk and protein" is described as a keyword in DEFINITION from sequences including INV as DIVISION among pieces of sequence information registered in NCBI GenBank, a sequence in which a specific character string of product is described from CDS, or a sequence in which a character string specific to TISSUE TYPE is described from SOURCE.

In the present specification, the "artificial fibroin" means an artificially produced fibroin (artificial fibroin). The artificial fibroin may be a fibroin having an amino acid sequence different from or identical to an amino acid sequence of a naturally derived fibroin. The artificial fibroin can be produced by a known method, for example, a method described in WO 2019/194263 A.

The artificial fibroin may be a fibrous protein having a structure similar to that of a naturally derived fibroin, or may be fibroin having a sequence similar to a repetitive sequence of the naturally derived fibroin. The "sequence similar to a repetitive sequence of the fibroin" may be an actual sequence of a naturally derived fibroin, or may be a sequence similar thereto.

The "artificial fibroin" may be a fibroin whose amino acid sequence is modified based on a naturally derived fibroin (for example, a fibroin whose amino acid sequence is modified by modifying a cloned gene sequence of a naturally derived fibroin) or a fibroin obtained by artificially designing an amino acid sequence independently of a naturally derived fibroin (for example, a fibroin having a desired amino acid sequence by chemically synthesizing a nucleic acid encoding a designed amino acid sequence) as long as the artificial fibroin has an amino acid sequence specified in the present disclosure. Note that a fibroin obtained by modifying an amino acid sequence of the artificial fibroin is also included in the artificial fibroin as long as an amino acid sequence thereof is different from an amino acid sequence of a naturally derived fibroin. Examples of the artificial fibroin include an artificial silk fibroin (a fibroin obtained by modifying an amino acid sequence of a silk protein produced by silkworms) and an artificial spider silk fibroin (a fibroin obtained by modifying an amino acid sequence of a spider silk protein produced by a spider). The artificial fibroin preferably contains an artificial spider silk fibroin as a molding material, and more preferably consists of the artificial spider silk fibroin because the artificial spider silk fibroin is relatively easily fibrillated and has a high fiber forming ability.

The artificial fibroin according to the present embodiment may be a protein including a domain sequence represented by Formula 1: [(A)n motif-REP]m or Formula 2: [(A)n motif-REP]m-(A)n motif. An amino acid sequence (N-terminal sequence or C-terminal sequence) may be further added to any one or both of an N-terminal side and a C-terminal side of the domain sequence of the artificial fibroin. The N-terminal sequence and the C-terminal sequence are typically regions not containing repeats of amino acid motifs that are characteristic of a fibroin and each consist of an amino acid having about 100 residues, but are not limited thereto.

In the present specification, the "domain sequence" means an amino acid sequence represented by Formula 1: [(A)n motif-REP]m or Formula 2: [(A)n motif-REP]m-(A)n motif. Here, the (A)n motif represents an amino acid sequence mainly containing alanine residues, and the number of amino acid residues is 2 to 27. The number of amino acid residues in the (A)n motif may be an integer of 2 to 20, 4 to 27, 4 to 20, 8 to 20, 10 to 20, 4 to 16, 8 to 16, or 10 to 16. A ratio of the number of alanine residues to the total number of amino acid residues in the (A)n motif only needs to be 40% or more, and may be 60% or more, 70% or more, 80% or more, 83% or more, 85% or more, 86% or more, 90% or more, 95% or more, or 100% (which means that the (A)n motif consists only of alanine residues). At least seven of the plurality of (A)n motifs present in the domain sequence may consist only of alanine residues. The REP represents an amino acid sequence consisting of 2 to 200 amino acid residues. The REP may be an amino acid sequence consisting of 10 to 200 amino acid residues. m represents an integer of 2 to 300 and may be an integer of 10 to 300. The plurality of (A)n motifs may be the same amino acid sequence or different amino acid sequences. The plurality of REPs may be the same amino acid sequence or different amino acid sequences.

Specific examples of the artificial fibroin include an artificial fibroin derived from a major dragline silk protein produced in a major ampullate gland of a spider as described in WO 2019/194263 A (first artificial fibroin), an artificial fibroin containing a domain sequence in which the content of glycine residues is reduced (second artificial fibroin), an artificial fibroin containing a domain sequence in which the content of an (A)n motif is reduced (third artificial fibroin), an artificial fibroin in which the content of glycine residues and the content of an (A)n motif are reduced (fourth artificial fibroin), an artificial fibroin containing a domain sequence including a region locally having a high hydropathy index (fifth artificial fibroin), and an artificial fibroin containing a domain sequence in which the content of glutamine residues is reduced (sixth artificial fibroin). Definition of each of the first to sixth artificial fibroins is incorporated herein by reference with the content described in WO 2019/194263 A.

The artificial fibroin may include a tag sequence at one or both of an N-terminal and a C-terminal thereof. This makes it possible to isolate, immobilize, detect, and visualize the artificial fibroin.

Examples of the tag sequence include an affinity tag using specific affinity (binding property or affinity) for another molecule. Specific examples of the affinity tag include a histidine tag (His tag). The His-tag is a short peptide in which about 4 to 10 histidine residues are lined up and can be used for isolating the artificial fibroin by chelating metal chromatography because the His-tag has a property of specifically binding to a metal ion such as nickel. Specific examples of the tag sequence include an amino acid sequence set forth in SEQ ID NO: 8 (an amino acid sequence including a His-tag sequence and a hinge sequence).

In addition, a tag sequence such as glutathione-S-transferase (GST) that specifically binds to glutathione or a maltose binding protein (MBP) that specifically binds to maltose can also be used.

Furthermore, an "epitope tag" using an antigen-antibody reaction can also be used. By adding a peptide (epitope) exhibiting antigenicity as a tag sequence, an antibody to the epitope can bind thereto. Examples of the epitope tag include an HA (a peptide sequence of influenza virus hemagglutinin) tag, a myc tag, and a FLAG tag. By using the epitope tag, the artificial fibroin can be easily purified with high specificity.

Furthermore, a tag sequence that can be cleaved by a specific protease can also be used. By treating a protein adsorbed via the tag sequence with a protease, an artificial fibroin from which the tag sequence is cleaved can be collected.

Specific examples of the artificial fibroin include artificial fibroins presented in Table 1.

**[Table 1]**

| | Alanine residue content (%) | Glycine residue content (%) | Serine residue content (%) | Threonine residue content (%) | Tyrosine residue content (%) | Glutamine residue content (%) | Lysine residue content (%) |
|---|---|---|---|---|---|---|---|
| PRT799 (SEQ ID NO: 1) | 19.8 | 31.0 | 9.3 | 0 | 7.0 | 17.3 | 0.1 |
| PRT966 (SEQ ID NO: 2) | 19.7 | 31.2 | 9.4 | 0 | 7.1 | 0 | 0 |
| PRT918 (SEQ ID NO: 3) | 19.5 | 30.9 | 9.7 | 0 | 7.0 | 0 | 0 |
| PRT313 (SEQ ID NO: 4) | 25.7 | 36.0 | 8.9 | 0 | 6.4 | 8.2 | 0 |
| PRT1000 (SEQ ID NO: 5) | 19.4 | 30.8 | 9.6 | 0 | 7.0 | 0 | 0 |
| PRT1001 (SEQ ID NO: 6) | 19.3 | 30.7 | 9.6 | 0 | 6.9 | 0 | 0 |
| PRT587 (SEQ ID NO: 7) | 19.7 | 31.1 | 9.4 | 0 | 7.1 | 17.3 | 0 |
| PRT2882 (SEQ ID NO: 9) | 16.4 | 28.2 | 11.8 | 0 | 2.8 | 0 | 0 |

The artificial fibroin may be an artificial fibroin having at least two or more of the characteristics of the first artificial fibroin, the second artificial fibroin, the third artificial fibroin, the fourth artificial fibroin, the fifth artificial fibroin, and the sixth artificial fibroin.

A molecular weight of the artificial fibroin is not particularly limited, and may be, for example, 2 kDa or more and 700 kDa or less The molecular weight of the artificial fibroin according to the present embodiment may be, for example, 2 kDa or more, 3 kDa or more, 4 kDa or more, 5 kDa or more, 6 kDa or more, 7 kDa or more, 8 kDa or more, 9 kDa or more, 10 kDa or more, 20 kDa or more, 30 kDa or more, 40 kDa or more, 50 kDa or more, 60 kDa or more, 70 kDa or more, 80 kDa or more, 90 kDa or more, or 100 kDa or more, and may be 700 kDa or less, 600 kDa or less, 500 kDa or less, 400 kDa or less, less than 360 kDa, 300 kDa or less, or 200 kDa or less.

As described above, in a case where a molded body having excellent flexibility is obtained using a synthetic polymer obtained by the production method, it is preferable to use a polypeptide having a relatively low molecular weight as component (A) when component (B1) is a compound having a low molecular weight. However, when the polypeptide has a low molecular weight (for example, 30 kDa or less), a yield may decrease during purification in preparation thereof. In particular, when a method for purifying a hydrophobic protein is used, a problem such as a decrease in purity may occur. Therefore, in the production method, for example, a protein (PRT2882) having an amino acid sequence in which a hydrophobic tag (for example, GFILGFIL in SEQ ID NO: 9) is introduced at an N-terminal or a C-terminal as set forth in SEQ ID NO: 9 may be used. Since a low-molecular-weight recombinant protein having an amino acid sequence including such a hydrophobic tag sequence has a high yield and a high purity at the time of purification thereof, a polymer compound having a low content of impurities can be obtained by the above-described production method. Therefore, in a molded body using such a polymer compound as a molding material, advantages such as obtaining better mechanical properties and the like can be advantageously ensured.

In the artificial fibroin according to the present embodiment, one or more of amino acid residues constituting the REP may be hydrophobic amino acid residues. That is, the REP preferably contains a hydrophobic amino acid residue. The hydrophobic amino acid residue means an amino acid residue having a positive hydrophobicity index. As the hydrophobicity index (hereinafter, also referred to as "HI") of the amino acid residue, a known index known index (Hydropathy index: Kyte J, & Doolittle R (1982)" A simple method for displaying the hydropathic character of a protein", J. Mol. Biol., 157, pp. 105-132) is used. Examples of the hydrophobic amino acid residue include isoleucine (HI: 4.5), valine (HI: 4.2), leucine (HI: 3.8), phenylalanine (HI: 2.8), methionine (HI: 1.9), and alanine (HI: 1.8).

In the artificial fibroin according to the present embodiment, the domain sequence preferably has an amino acid sequence corresponding to an amino acid sequence in which a cysteine residue is inserted into the REP as compared with a naturally derived fibroin.

The domain sequence preferably has an amino acid sequence corresponding to an amino acid sequence in which a cysteine residue is inserted at a position adjacent to a glycine residue, a serine residue, or an alanine residue in the REP, and more preferably has an amino acid sequence corresponding to an amino acid sequence in which a cysteine residue is inserted at a position adjacent to a glycine residue in the REP. A cysteine residue in the REP may be located between a glycine residue, a serine residue, or an alanine residue and a glycine residue, a serine residue, or an alanine residue, or may be located between a serine residue and a glycine residue.

The domain sequence preferably has an amino acid sequence corresponding to an amino acid sequence in which a cysteine residue is inserted at a position adjacent to a hydrophobic amino acid residue in the REP. In this case, the hydrophobic amino acid residues are immobilized by a hydrophobic interaction between molecules. The cysteine residue in the REP may be located adjacent to a hydrophobic amino acid residue, may be located between the hydrophobic amino acid residue and an amino acid residue other than the hydrophobic amino acid residue, may be located between the hydrophobic amino acid residue and a glycine residue, a serine residue, or an alanine residue, or may be located between the hydrophobic amino acid residue and a glycine residue. The hydrophobic amino acid residue may be one selected from the group consisting of an isoleucine residue, a valine residue, a leucine residue, a phenylalanine residue, a methionine residue, and an alanine residue.

The domain sequence may have an amino acid sequence corresponding to an amino acid sequence in which a cysteine residue is inserted into an REP located in the vicinity of an N-terminal and/or a C-terminal of the domain sequence as compared with naturally derived fibroin. In this case, a molecular chain can be lengthened. In the present specification, the REP located in the vicinity of the N-terminal of the domain sequence means an REP located at positions 1 to 3 from the N-terminal of the domain sequence. For example, a cysteine residue may be located in an REP located at positions 1 and 2 from the N-terminal of the domain sequence. In the present specification, the REP located in the vicinity of the C-terminal of the domain sequence means an REP located at positions 1 to 3 from the C-terminal of the domain sequence. For example, the cysteine residue may be located in an REP located at positions 1 and 2 from the C-terminal of the domain sequence. The cysteine residue is preferably located in an REP located closest to the N-terminal side and/or closest to the C-terminal side of the domain sequence.

The domain sequence may have an amino acid sequence corresponding to an amino acid sequence in which a cysteine residue is inserted into the center or the vicinity of the center thereof in an REP as compared with naturally derived fibroin. In the present specification, the vicinity of the center of the amino acid sequence in the REP indicates positions 1 to 5 from an amino acid residue located in the center of the REP (an amino acid residue on an N-terminal side when two amino acid residues located in the center are present) toward the N-terminal side, or positions 1 to 5 from the amino acid residue located in the center of the REP (an amino acid residue on a C-terminal side when two amino acid residues located in the center are present). For example, the cysteine residue may be located in the center of the REP, or may be located at positions 1 to 3 or 1 and 2 from the amino acid residue located in the center of the REP toward the N-terminal side or the C-terminal side. Here, for example, when a first segment containing a polypeptide skeleton in which cysteine residues are inserted so as to be located one by one on the N-terminal side and the C-terminal side of the domain sequence is used, a synthetic polymer in which such a first segment and a second segment are alternately linked so as to be located one by one can be obtained. A molded body (for example, a fiber, a film, or a gel) obtained using this synthetic polymer can be expected to have improved elongation. When a first segment containing a polypeptide skeleton in which a cysteine residue is inserted so as to be located more centrally than the N-terminal side and the C-terminal side of the domain sequence is used, a synthetic polymer in which a second segment is linked to such a first segment can be expected to have improved solubility in a solvent.

A hydropathy index of an REP in the artificial fibroin may be, for example, -0.80, -0.70, -0.06 or more, - 0.50 or more, -0.40 or more, -0.30 or more, -0.20 or more, -0.10 or more, 0.00 or more, 0.10 or more, 0.20 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. An upper limit of the hydropathy index of an REP is not particularly limited, and may be 1.0 or less or 0.7 or less.

In the present specification, the "hydropathy index of an REP" is a value calculated by the following method. The hydropathy index of an REP is calculated as e/f in a case where in all REPs included in a sequence excluding a sequence from an (A)ₙ motif located at the most C-terminal side to the C-terminal of the domain sequence from the domain sequence in a fibroin including a domain sequence represented by Formula 1: [(A)ₙ motif-REP]ₘ or Formula 2: [(A)ₙ motif -REP]ₘ-(A)ₙ motif, the sum of the hydropathy indices of each of amino acid residues in this region is represented by e, and the total number of amino acid residues in all REPs excluding the sequence from the (A)ₙ motif located at the most C-terminal side to the C-terminal of the domain sequence from the domain sequence and further excluding the (A)ₙ motifs is represented by f. In the calculation of the hydropathy index of an REP, a reason for targeting the "sequence excluding the sequence from the (A)ₙ motif located at the most C-terminal side to the C-terminal of the domain sequence from the domain sequence" is similar to the reason described above.

The domain sequence may have an amino acid sequence corresponding to an amino acid sequence in which one or more and less than 16 cysteine residues are inserted into an REP as compared with a naturally derived fibroin. That is, the total number of cysteine residues corresponding to the cysteine residues inserted into the REP may be one or more and less than 16. The total number of cysteine residues corresponding to the cysteine residues inserted into the REP may be 1 or more and 12 or less, 1 or more and 10 or less, 1 or more and 8 or less, 1 or more and 6 or less, or 2 or more and 4 or less. The number of cysteine residues per REP in the domain sequence may be, for example, 1 to 3, 1 or 2, or 1.

The total number of cysteine residues in the artificial fibroin according to the present embodiment may be 1 or more and less than 16, 1 or more and 12 or less, 1 or more and 10 or less, 1 or more and 8 or less, 1 or more and 6 or less, or 2 or more and 4 or less.

The artificial fibroin according to the present embodiment may be obtained by, for example, modification of an amino acid sequence corresponding to substitution, deletion, insertion, and/or addition of one or more amino acid residues in addition to modification related to the cysteine residue in the REP described above as compared with a naturally derived fibroin.

The artificial fibroin according to the present embodiment preferably has an amino acid sequence corresponding to an amino acid sequence in which a cysteine residue is inserted.

A method for preparing component (B1) may be a method including esterifying maleic anhydride with at least one compound selected from the group consisting of a polyether, a polyester, and a polycarbonate. At this time, an acid or a base may be used, or the reaction may be allowed to proceed in the absence of a solvent. In the preparation of component (B1), introduction of a protecting group into a polyether or the like is not particularly necessary, and a simple method can be adopted. Purification may be performed, for example, by performing washing with a solvent such as cyclopentyl methyl ether, tetrahydrofuran, ethyl acetate, or ethanol to remove impurities.

A second embodiment of the polymer compound production method includes subjecting a polypeptide having at least one mercapto group, and at least one compound selected from the group consisting of a polyether, a polyester, and a polycarbonate and having two structures represented by the following general formula (2) (hereinafter, also referred to as component (B2)) to a heating reaction in dimethyl sulfoxide in the presence of a base, a reducing agent, and a polymerization inhibitor (hereinafter, also referred to as component (E)). In the following general formula (2), R¹ represents a hydrogen atom or a methyl group. The heating reaction is a reaction between component (A) and component (B2), and is performed, for example, by performing heating to 50°C or higher. Note that a heating timing is not limited to a timing after components (A) to (E) are mixed, and for example, components (A) to (E) may be added using dimethyl sulfoxide heated in advance, heating may be started after components (C), (D), and (E) are added to dimethyl sulfoxide and components (A) and (B) may be added and caused to react after a predetermined temperature is reached, or heating may be started after one of components (A) and (B) and components (C), (D), and (E) are added, and the remaining one of components (A) and (B) may be added and caused to react after a predetermined temperature is reached.

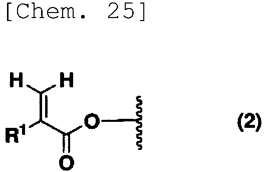

In the following description, matters common to the first embodiment will not be described, and different points will be described. For the polypeptide having at least one mercapto group, the base, and the reducing agent in the second embodiment, the description for component (A), component (C), and component (D) in the first embodiment can be applied.

At least one compound (component (B2)) selected from the group consisting of a polyether, a polyester, and a polycarbonate and having two structures represented by general formula (2) is a compound capable of reacting with a mercapto group of the above-described polypeptide and capable of introducing a polyether structure, a polyester structure, or a polycarbonate structure into the polypeptide by having the structure represented by formula (1).

Examples of the polyether having two structures represented by formula (2) include polyethylene glycol, polytetramethylene glycol, polypropylene glycol, and an ethylene glycol-propylene glycol copolymer. Examples of the polyester having two structures represented by formula (2) include polylactic acid, polyglycolic acid, polybutylene succinate, polycaprolactone, and polyhydroxyalkanoates including a 3-hydroxybutanoic acid-3-hydroxyhexanoic acid copolymer and the like. Examples of the polycarbonate having two structures represented by formula (2) include aliphatic polycarbonates such as polyethylene carbonate, polypropylene carbonate, and polytrimethylene carbonate. Since these compounds are compounds that are easier in molecular motion than the above-described polypeptide, when each of these compounds is a compound as described above, flexibility of a molded body containing the resulting polymer compound can be further improved.

For the blending amount of component (B2) and the like, the description of component (B1) in the first embodiment can be applied by replacing the description with that of component (B2).

The polymerization inhibitor (component (E)) in the above-described production method is a compound that inhibits self-polymerization of component (B2). Examples of component (E) include hydroquinone, methoquinone, 4-tert-butylpyrocatechol, tert-butylhydroquinone, 1,4-benzoquinone, dibutylhydroxytoluene, mequinol, phenothiazine, and 1,1-diphenyl-2-picrylhydrazyl.

The content of component (E) may be, for example, 0.01 parts by mass or more, 0.05 parts by mass or more, 0.1 parts by mass or more, or 0.2 parts by mass or more with respect to 100 parts by mass of the compound (component (B2)). By setting the content of the polymerization inhibitor within the above range, it is possible to suppress initiation of radical polymerization of the structure represented by general formula (2) and to make the reaction between the structure represented by general formula (2) and a mercapto group of the polypeptide more dominant. The content of component (E) may be, for example, 25 parts by mass or less, 10 parts by mass or less, 5 parts by mass or less, or 1 part by mass or less with respect to 100 parts by mass of component (B2). By setting the content of component (E) within the above range, inhibition of the reaction by component (E) in the system can be further suppressed.

A method for preparing component (B2) may be a method including esterifying (meth)acrylic acid, anhydrous (meth)acrylic acid, (meth)acryloyl chloride, and at least one compound selected from the group consisting of a polyether, a polyester, and a polycarbonate. In the preparation of component (B2), introduction of a protecting group into a polyether or the like is not particularly necessary, and a simple method can be adopted. Purification was performed by removing an organic salt by thermal extraction with tetrahydrofuran, and further adding n-hexane to perform reprecipitation.

A polymer compound synthesized by the above-described production method has a structure in which a polypeptide moiety and at least one structural unit selected from the group consisting of a polyether structure, a polyester structure, and a polycarbonate structure directly bind to each other by a structure represented by the following general formula (3), (4), or (5) such that the polypeptide moiety binds to S of the following general formula (3), (4), or (5). In general formulas (3) and (4), M represents any one of H (hydrogen atom), Na (sodium atom), K (potassium atom), NHEt₃, and NHEtiPr₂, and in general formula (5), R¹ represents H or Me. The Me represents a methyl group, the Et represents an ethyl group, and the iPr represents an isopropyl group.

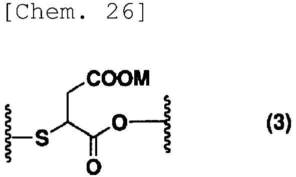

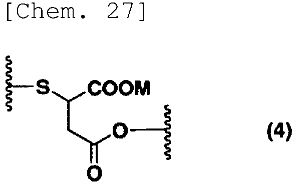

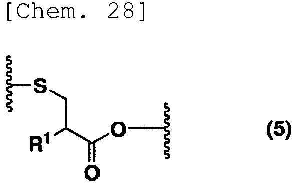

The above-described polymer compound has a polypeptide and at least one structural unit selected from the group consisting of a polyether structure, a polyester structure, and a polycarbonate structure, and therefore can also be referred to as a block copolymer. When a structural unit containing a polypeptide is represented by A and a structural unit containing at least one of a polyether structure, a polyester structure, and a polycarbonate structure is represented by B, the above-described polymer compound only needs to have at least one A and at least one B. The above-described polymer compound may be, for example, an A-B diblock copolymer, an A-B-A triblock copolymer, an A-B-A-B tetrablock copolymer, an A-B-A-B-A pentablock copolymer, or the like, or may be a copolymer such as A-A-B-B-B-B-A-B-B-A-A.

The polymer compound may be a linear polymer or a comb polymer, and may have a three-dimensional network structure. The polymer compound is preferably a linear polymer or a comb polymer from a viewpoint of further improving flexibility of a molded body containing the polymer compound, and the polymer compound preferably has a three-dimensional network structure from a viewpoint of improving elastic modulus of the molded body.

The position of at least one structural unit selected from the group consisting of a polyether structure, a polyester structure, and a polycarbonate structure to be introduced with respect to the polypeptide moiety is not particularly limited, and may be an end of the polypeptide moiety or may be a position other than the end of the polypeptide moiety, but is preferably the end of the polypeptide moiety from a viewpoint of ease of production of a polymer compound.

The polymer compound synthesized by the above-described production method can be used as a molding material. The shape of a molded body using the polymer compound as a molding material is not particularly limited, and may be, for example, a film or a fiber.

One aspect of the present disclosure provides a method for producing a polymer compound solution, the method including dissolving a polymer compound obtained by the above-described production method in a solvent. A polymer compound solution obtained by such a method can be used as a so-called dope solution in preparing a molded body. Examples of a solvent used for preparing the dope solution include formic acid, dimethyl sulfoxide, N,N-dimethylformamide, N,N-dimethylacetamide, hexafluoro-2-propanol, and N-methyl-2-pyrrolidone. Note that, in the above-described production method, a target polymer compound is obtained in a state of being dissolved in dimethyl sulfoxide (as a dimethyl sulfoxide solution) by the above-described heating reaction. Therefore, the above method for producing a polymer compound solution includes a step of dissolving a polymer compound extracted from dimethyl sulfoxide by, for example, a known extraction method or separation method in a predetermined solvent.

A polymer film may be produced by a method including a step of molding a film from a polymer compound solution obtained by the above-described method for producing a polymer compound solution. The molding of the film may be, for example, cast molding.

A polymer fiber may be produced by a method including a step of spinning a polymer compound solution obtained by the above-described method for producing a polymer compound solution.

Note that a dimethyl sulfoxide solution of a polymer compound obtained by the heating reaction in the above-described polymer compound production method can be used as a dope to be used for producing a film or a fiber as it is. A polymer film can be produced more easily and at low cost. The molding of a film may also be, for example, cast molding.

One aspect of the present disclosure provides a method for producing a film, the method including: subjecting a polypeptide having at least one mercapto group and at least one compound selected from the group consisting of a polyether, a polyester, and a polycarbonate and having two structures represented by the following general formula (1) to a heating reaction in dimethyl sulfoxide in the presence of a base and a reducing agent to obtain a polymer compound solution (a dimethyl sulfoxide solution of a polymer compound); and molding a film from the polymer compound solution. In general formula (1), M represents any one of H, Na, K, NHEt₃, and NHEtiPr₂.

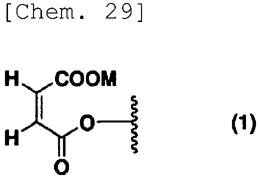

One aspect of the present disclosure provides a method for producing a film, the method including: subjecting a polypeptide having at least one mercapto group and at least one compound selected from the group consisting of a polyether, a polyester, and a polycarbonate and having two structures represented by the following general formula (2) to a heating reaction in dimethyl sulfoxide in the presence of a base, a reducing agent, and a polymerization inhibitor to obtain a polymer compound solution (a dimethyl sulfoxide solution of a polymer compound); and molding a film from the polymer compound solution. In general formula (2), R¹ represents a hydrogen atom or a methyl group.

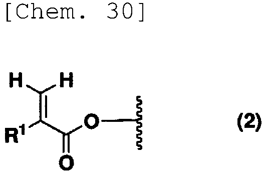

One aspect of the present disclosure provides a method for producing a fiber, the method including: subjecting a polypeptide having at least one mercapto group and at least one compound selected from the group consisting of a polyether, a polyester, and a polycarbonate and having two structures represented by the following general formula (1) to a heating reaction in dimethyl sulfoxide in the presence of a base and a reducing agent to obtain a polymer compound solution (a dimethyl sulfoxide solution of a polymer compound); and spinning the polymer compound solution. In general formula (1), M represents any one of H, Na, K, NHEt₃, and NHEtiPr₂.

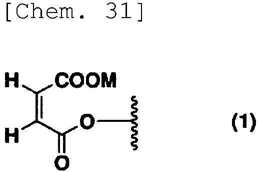

One aspect of the present disclosure provides a method for producing a fiber, the method including: subjecting a polypeptide having at least one mercapto group and at least one compound selected from the group consisting of a polyether, a polyester, and a polycarbonate and having two structures represented by the following general formula (2) to a heating reaction in dimethyl sulfoxide in the presence of a base, a reducing agent, and a polymerization inhibitor to obtain a polymer compound solution (a dimethyl sulfoxide solution of a polymer compound); and spinning the polymer compound solution. In general formula (2), R¹ represents a hydrogen atom or a methyl group.

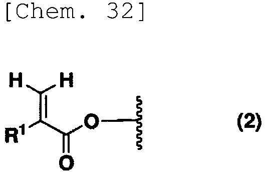

One aspect of the present disclosure provides a film containing a polymer compound, in which the polymer compound has a structure in which a polypeptide moiety and at least one structural unit selected from the group consisting of a polyether structure, a polyester structure, and a polycarbonate structure directly bind to each other by a structure represented by the following formula (3), (4), or (5) such that the polypeptide moiety binds to S of the following formula (3), (4), or (5), and the film has an elongation of 400% or more and a ratio of breaking strength to yield point strength of more than 1. In general formulas (3) and (4), M represents any one of H, Na, K, NHEt₃, and NHEtiPr₂, and in general formula (5), R¹ represents H or Me.

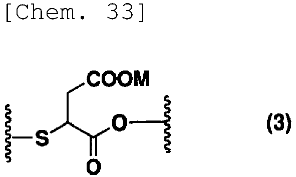

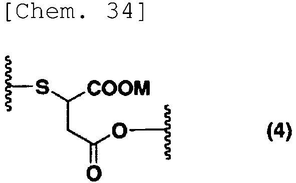

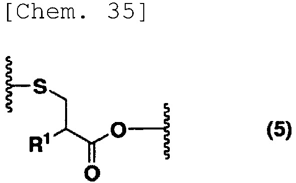

One aspect of the present disclosure provides a fiber containing a polymer compound, in which the polymer compound has a structure in which a polypeptide moiety and at least one structural unit selected from the group consisting of a polyether structure, a polyester structure, and a polycarbonate structure directly bind each other by a structure represented by the following formula (3), (4), or (5) such that the polypeptide moiety binds to S of the following formula (3), (4), or (5), and the fiber has an elongation of 400% or more and a ratio of breaking strength to yield point strength of more than 1. In general formula (5), R¹ represents a hydrogen atom or a methyl group.

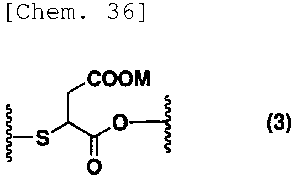

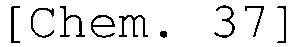

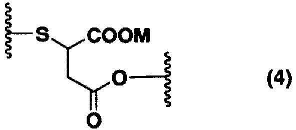

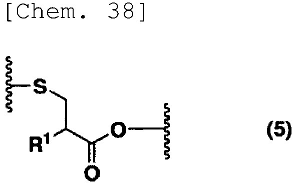

In the film and the fiber, the ratio of breaking strength to yield point strength (value of breaking strength [MPa]/yield point strength [MPa]) is more than 1, but may be, for example, 1.1 or more, 1.2 or more, 1.3 or more, 1.4 or more, or 1.5 or more. In the film and the fiber, the ratio of breaking strength to yield point strength (value of breaking strength [MPa]/yield point strength [MPa]) may be, for example, 3 or less or 2 or less.

The polymer compound according to the present embodiment can be used as an adhesive for bonding an adherend or a coating agent for laminating a coating layer on a base material in a solution state, an aqueous dispersion state, a film state, or a powder state containing the polymer compound as a main component. The solution-state adhesive or the solution-state coating agent, the water-dispersible adhesive or the water-dispersible coating agent, the film-shaped adhesive or the film-shaped coating agent, the powdery adhesive or the powdery coating agent can be obtained by, for example, the following production method.

The solution-state adhesive or the solution-state coating agent containing the polymer compound can be produced by a method including a step of dissolving a polymer compound obtained by the present embodiment method in a solvent. According to such a method, unlike a conventional solution-state adhesive or solution-state coating agent containing a petroleum-derived component, a biodegradable solution-state adhesive or solution-state coating agent can be easily obtained. Note that, in the above-described polymer compound production method according to the present embodiment, a target polymer compound is obtained in a state of being dissolved in dimethyl sulfoxide (as a dimethyl sulfoxide solution) by the heating reaction described above. Therefore, a method for producing the solution-state adhesive or the coating agent according to the present embodiment includes a step of dissolving a polymer compound extracted from dimethyl sulfoxide by, for example, a known extraction method or separation method in a predetermined solvent. In addition, the solution-state adhesive or the solution-state coating agent according to the present embodiment may contain a dimethyl sulfoxide solution of a polymer compound obtained by the heating reaction in the polymer compound production method.

Examples of a solvent used in production of the solution-state adhesive or the solution-state coating agent include an aqueous medium such as water in which a polymer compound can be dissolved, a basic aqueous solution, an acidic aqueous solution, or a neutral aqueous solution containing an inorganic salt, and an organic solvent. Examples of the organic solvent include formic acid, dimethyl sulfoxide (DMSO), hexafluoroisopropanol (HFIP), N,N-dimethylformamide (DMF), N,N-dimethylacetamide (DMAc), N-methylpiperidone (NMP), and dihydrolevoglucosenone.

The polymer compound contained in the solution-state adhesive or the solution-state coating agent is obtained by the production method according to the present embodiment, and is not particularly limited as long as the polymer compound can be dissolved in the above-described aqueous medium or organic solvent. An adhesive or a coating agent in which the polymer compound is dissolved in an aqueous medium has an advantage that handling properties and the like are superior to those of an adhesive or a coating agent in which the polymer compound is dissolved in an organic solvent. Note that, in the polymer compound according to the present embodiment, since a molecule capable of plasticizing a protein binds to the protein, the polymer compound exhibits higher water solubility than the protein. Therefore, an aqueous adhesive or an aqueous coating agent in which the polymer compound is dissolved in an aqueous medium can have a higher protein content than an aqueous adhesive or an aqueous coating agent in which a protein is simply dissolved in an aqueous medium. Therefore, when an aqueous adhesive or an aqueous coating agent in which the polymer compound is dissolved in an aqueous medium is used, it can be expected that adhesive strength of the adhesive or the coating agent to an adhesion surface of an adherend or a laminate surface described later is improved as compared with a case of using an aqueous adhesive or an aqueous coating agent in which a protein is dissolved in an aqueous medium.

A concentration of the polymer compound in a solution-state adhesive or a solution-state coating agent is appropriately determined based on a solubility of the polymer compound in a solvent or the like. The concentration may be, for example, 5 to 40% by mass, 10 to 35% by mass, 15 to 20% by mass, or 20 to 25% by mass. In addition, the solution-state adhesive or the solution-state coating agent may contain, as necessary, a component other than the polymer compound, such as various additives contained in a known solution-state adhesive or solution-state coating agent. An upper limit value of the concentration may be, for example, less than 30% by mass, 28% by mass or less, 25% by mass or less, or 23% by mass or less.

The solution-state adhesive containing the polymer compound is used for producing an adhesive body in which a plurality of adherends is bonded to each other. For example, the solution-state adhesive is interposed between a plurality of adherends to be bonded, then a solvent in the solution-state adhesive is removed to solidify the polymer compound, whereby the adherends can be bonded to each other to obtain an adhesive body. According to such a method, since an adhesive body is obtained using a biodegradable adhesive, there is an advantage that an adhesive body capable of reducing an environmental load at the time of disposal can be easily produced. Note that a material of the adherend is not particularly limited as long as the adherends can be bonded to each other with a solution-state adhesive containing the polymer compound, and the adherend may be an organic substance (for example, a cellulose product such as paper or wood, a synthetic resin, or a protein product) or an inorganic substance (a metal or a non-metal such as glass).

A specific method for producing the adhesive body using the solution-state adhesive is not limited at all. For example, the solution-state adhesive is put on an adhesion surface by applying or dropping the solution-state adhesive to an adhesion surface of at least one of adherends to be bonded to each other, or by bringing the adhesion surfaces of the adherends into contact with a liquid surface of the solution-state adhesive or immersing the adhesion surfaces in the liquid surface, then the adherends are superimposed on each other or caused to butt against each other at the adhesion surfaces, whereby the solution-state adhesive may be interposed between the plurality of adherends. In addition, when a solvent in the solution-state adhesive interposed between the plurality of adherends is removed to solidify the polymer compound, for example, heating, air drying, or natural drying may be performed in a state where pressure is applied to the plurality of adherends from at least one of a superimposing direction and a butting direction of the adhesion surfaces such that the superimposed or butted adhesion surfaces are brought into close contact with each other. Of course, when bonding is possible without applying such pressure, it is not necessary to apply pressure.

The solution-state coating agent containing the polymer compound is used for producing a laminate in which a coating layer is laminated on the whole or a part of a surface of a base material. For example, the solution-state coating agent is supplied to at least a part of a surface of a base material on which a coating layer is to be laminated, the surface part of the base material is coated with the solution-state coating agent, and then a solvent in the solution-state adhesive is removed to solidify the polymer compound. This makes it possible to obtain a laminate by laminating the coating layer on at least a part of the surface of the base material. Also by such a method, a laminate can be obtained using a biodegradable coating agent, and therefore there is an advantage that a laminate capable of reducing an environmental load at the time of disposal can be easily produced. Note that a material of the base material is not particularly limited as long as a solution-state coating agent containing the polymer compound can be bonded to the base material, and the base material may be similar to an adherend to be bonded with the solution-state adhesive described above.

A specific method for producing a laminate using the solution-state coating agent is also not limited at all. For example, by applying or dropping the solution-state coating agent to at least a part of a surface of the base material, bringing at least a part of the surface of the base material into contact with a liquid surface of the solution-state coating agent, or immersing at least a part of the surface of the base material in the liquid surface, the solution-state coating agent may be supplied to at least a part of the base material so as to form a layer having a predetermined thickness to perform coating. When the polymer compound in the coating agent layer (solution-state coating agent) laminated on the surface of the base material is solidified, for example, the coating agent layer on the surface of the base material may be heated, air-dried, or naturally dried to remove the solvent in the coating agent layer. Note that, at this time, a predetermined pressing body (pressurizing body) may be placed on the coating agent layer so as to cover the entire surface of the coating agent layer, as necessary, and the coating agent layer may be pressed (pressurized) against the surface of the base material such that the coating agent layer is in close contact with the surface of the base material. At this time, it is preferable to prevent the pressing body from being bonded to the solution-state coating agent. For example, by applying a release agent to a contact surface of the pressing body with the coating agent layer, attaching release paper to the contact surface, performing a surface treatment such that the coating agent is not bonded to the contact surface, or using a material that is not bonded to the solution-state coating agent as the pressing body, the adhesion of the solution-state coating agent to the pressing body can be prevented.

The water-dispersible adhesive or the water-dispersible coating agent containing the polymer compound can be produced by a method including a step of dispersing a polymer compound obtained by the present embodiment method in an aqueous medium. According to such a method, unlike a conventional water-dispersible adhesive or water-dispersible coating agent containing a petroleum-derived component, a biodegradable water-dispersible adhesive or water-dispersible coating agent can be easily obtained. As described above, in the polymer compound production method according to the present embodiment, a target polymer compound is obtained in a state of being dissolved in dimethyl sulfoxide (as a dimethyl sulfoxide solution). Therefore, a method for producing the water-dispersible adhesive or the water-dispersible coating agent according to the present embodiment includes a step of dispersing a polymer compound extracted from dimethyl sulfoxide by, for example, a known extraction method or separation method in a predetermined aqueous medium.

Examples of the aqueous medium used for producing the water-dispersible adhesive or the water-dispersible coating agent include water in which the polymer compound can be dispersed, a basic aqueous solution, an acidic aqueous solution, and an aqueous solution containing an inorganic salt.

The polymer compound contained in the water-dispersible adhesive or the water-dispersible coating agent is obtained by the production method according to the present embodiment, and is not particularly limited as long as the polymer compound can be dispersed in the aqueous medium described above. The content of the polymer compound in the water-dispersible adhesive or the water-dispersible coating agent is appropriately determined in consideration of adhesiveness of the water-dispersible adhesive to an adherend, adhesiveness or fixability of the water-dispersible coating agent to a base material, and the like. In addition, the water-dispersible adhesive and the water-dispersible coating agent may contain, as necessary, a component other than the polymer compound, such as various additives contained in a known water-dispersible adhesive or water-dispersible coating agent.

Note that, in the polymer compound according to the present embodiment, since a molecule capable of plasticizing a protein binds to the protein, the polymer compound exhibits higher affinity for an aqueous medium containing water than the protein. Therefore, in such a water-dispersible adhesive or water-dispersible coating agent containing the polymer compound, the content (dispersion amount) of a protein can be increased and the polymer compound can be uniformly dispersed in an aqueous medium as compared with a water-dispersible adhesive or a water-dispersible coating agent in which a protein is simply dispersed in an aqueous medium. Therefore, when a water-dispersible adhesive or a water-dispersible coating agent in which the polymer compound is dispersed in an aqueous medium is used, it can be expected that adhesive strength of the adhesive or the coating agent to an adhesion surface of an adherend or a laminate surface described later is improved as compared with a case of using a water-dispersible adhesive or a water-dispersible coating agent in which a protein is dispersed in an aqueous medium.

The water-dispersible adhesive containing the polymer compound is used for producing an adhesive body in which a plurality of adherends is bonded to each other. For example, the water-dispersible adhesive is interposed between a plurality of adherends to be bonded, then an aqueous medium in the water-dispersible adhesive is removed to solidify the polymer compound, whereby adherends can be bonded to each other to obtain an adhesive body. According to such a method, since an adhesive body is obtained using a biodegradable adhesive, there is an advantage that an adhesive body capable of reducing an environmental load at the time of disposal can be easily produced. Note that a material of the adherend is not particularly limited as long as the adherend can be bonded with the water-dispersible adhesive containing the polymer compound, and the adherend may be similar to an adherend to be bonded with the solution-state adhesive containing the polymer compound.

A specific method for producing the adhesive body using the water-dispersible adhesive is not also limited at all. For example, when the water-dispersible adhesive is interposed between a plurality of adherends to be bonded, a similar method to the method used when the solution-state adhesive is interposed between a plurality of adherends can be adopted. In addition, also when the aqueous medium in the water-dispersible adhesive interposed between the adherends is removed to solidify the polymer compound, a similar method to the method used when the solvent in the solution-state adhesive interposed between the adherends is removed to solidify the polymer compound can be adopted.

The water-dispersible coating agent containing the polymer compound is used for producing a laminate in which a coating layer is laminated on the whole or a part of a surface of a base material. For example, the water-dispersible coating agent is supplied to at least a part of a surface of a base material on which a coating layer is to be laminated, the surface part of the base material is coated with the solution-state coating agent, and then the aqueous medium in the water-dispersible adhesive is removed to solidify the polymer compound. This makes it possible to obtain a laminate by laminating the coating layer on at least a part of the surface of the base material. Also by such a method, a laminate can be obtained using a biodegradable coating agent, and therefore there is an advantage that a laminate capable of reducing an environmental load at the time of disposal can be easily produced. Note that a material of the base material is not particularly limited as long as the water-dispersible coating agent containing the polymer compound can be bonded to the base material, and the base material may be similar to a base material on which a coating layer is formed with the solution-state coating agent described above.

A specific method for producing a laminate using the water-dispersible coating agent is not also limited at all. For example, when the water-dispersible coating agent is supplied to at least a part of the surface of the base material, a similar method to the method used when the above-described solution-state coating agent is supplied to the surface of the base material can be adopted. In addition, when the aqueous medium in the water-dispersible coating agent supplied to the surface of the base material is removed to solidify the polymer compound, a method similar to the method used when the solvent in the solution-state coating agent supplied to the surface of the base material is removed to solidify the polymer compound can be adopted.

The film-shaped adhesive or the film-shaped coating agent containing the polymer compound can be produced by a method including a step of molding a film from a polymer compound solution in which a polymer compound obtained by the present embodiment method is dissolved. According to such a method, unlike a conventional film-shaped adhesive or film-shaped coating agent containing a petroleum-derived component, a biodegradable film-shaped adhesive or film-shaped coating agent having can be easily obtained. Note that a method for molding the film may be known cast molding.

As a film forming solution used for producing the film-shaped adhesive or the film-shaped coating agent, for example, a polymer compound solution used as the solution-state adhesive or the solution-state coating agent is suitably used. Therefore, such a film forming solution may be a dimethyl sulfoxide solution of a polymer compound obtained by the polymer compound production method according to the present embodiment, or may be a solution obtained by dissolving a polymer compound extracted from dimethyl sulfoxide by, for example, a known extraction method or separation method in a predetermined solvent. Note that when the polymer compound solution is cast-molded, a known method and a known condition similar to those when a protein solution is cast-molded can be adopted.

The film-shaped adhesive containing the polymer compound is used for producing an adhesive body in which a plurality of adherends is bonded to each other. For example, in a state where a film-shaped adhesive is interposed between a plurality of adherends to be bonded, the film-shaped adhesive is swollen or heated to be softened, and then the film-shaped adhesive is cured in a state of being brought into pressure contact with the adherends, whereby the adherends can be bonded to each other to obtain an adhesive body. In addition, the film-shaped adhesive may be swollen or heated to be softened in advance, interposed between a plurality of adherends, and then cured in a state of being brought into pressure contact with the adherends. According to these methods, since an adhesive body is obtained using a biodegradable adhesive, there is an advantage that an adhesive body capable of reducing an environmental load at the time of disposal can be easily produced. Note that the adherend may be any adherend as long as the adherend can be bonded with the film-shaped adhesive containing the polymer compound, and the adherend may be similar to an adherend to be bonded with the solution-state adhesive containing the polymer compound.

The film-shaped adhesive is softened when it absorbs moisture or is heated, and is cured by subsequent drying or cooling. In addition, when the polymer compound contained in the film-shaped adhesive has a property of exhibiting a shrinkage behavior by contact with water or heating particularly when being formed into a molded body, the film-shaped adhesive also shrinks by contact with water or heating. Therefore, when the film-shaped adhesive containing the polymer compound is interposed between adherends in a state of being swollen or heated to be softened, the film-shaped adhesive partially bites into a gap present on an adhesion surface of each of the adherends, and in particular, when the modified protein has the above-described shrinkage property, the film-shaped adhesive shrinks in a state of biting into the gap of the adhesion surface. When the film-shaped adhesive is cured in this state, it is considered that the film-shaped adhesive and the adherends are bonded to each other by an anchor effect, and the adherends are bonded to each other. In addition, the film-shaped adhesive exhibits sufficient flexibility by containing, as a main component, a polymer compound in which a molecule that plasticizes a protein binds to the protein. Therefore, such a film-shaped adhesive exhibits higher flexibility when being softened, and thus more sufficiently bites into the gap of the adhesion surface of each of the adherends, and as a result, it can be expected that higher adhesive strength can be obtained. Moreover, since the film-shaped adhesive has excellent flexibility, the film-shaped adhesive can be peeled off from the adherend after being bonded to the adherend, and further exhibits an excellent function of being reusable as the film-shaped adhesive. Note that when the film-shaped adhesive is reused, the film-shaped adhesive may be washed with water after being peeled off from the adherend. Note that it is also considered that adhesion of the film-shaped adhesive to the adherend is caused by hydrogen bonding between the adherend and the polymer compound.

When the film-shaped adhesive is swollen to be softened, an aqueous liquid such as water that is absorbed by the film-shaped adhesive and can shrink the film-shaped adhesive, or a solution or a dispersion containing water is suitably used. Then, for example, by causing the film-shaped adhesive to absorb water by dropping the above-described aqueous liquid or immersing a plurality of adherends in the aqueous liquid, the film-shaped adhesive may be swollen to be softened. In addition, when the film-shaped adhesive softened by swelling is cured, for example, a method for heating and drying, air-drying, or naturally drying the film-shaped adhesive in a state where the film-shaped adhesive is interposed between adherends can be adopted.

When the film-shaped adhesive is heated to be softened, the film-shaped adhesive may be heated before the film-shaped adhesive is interposed between adherends, or the whole of the adherends and the film-shaped adhesive may be heated after the film-shaped adhesive is interposed between the adherends. A heating temperature is not particularly limited as long as the heating temperature is a temperature at which the film-shaped adhesive can be softened and a protein contained in the film-shaped adhesive is not adversely affected (for example, not decomposed). When the film-shaped adhesive softened by heating is cured, a method for cooling the film-shaped adhesive with a cooling device or allowing the film-shaped adhesive to cool can be adopted.

In order to cause the film-shaped adhesive softened by swelling or heating to sufficiently bite into a gap of an adhesion surface of the adherend, it is desirable to bring an interface between the softened film-shaped adhesive and the adherend into close contact with each other. Therefore, for example, it is preferable to apply pressure to a plurality of adherends from at least one of an overlapping direction and a butting direction of the adhesion surfaces to cure the softened film-shaped adhesive while pressing the softened film-shaped adhesive against the adherends. Any general method can be adopted as the method for pressurizing the adherends here.

The film-shaped coating agent containing the polymer compound is used for producing a laminate in which a coating layer is laminated on the whole or a part of a surface of a base material. For example, in a state where the film-shaped coating agent is placed so as to cover at least a part of a surface of the base material, the film-shaped coating agent is swollen or heated to be softened, and then cured in a state of being brought into pressure contact with the surface of the base material, whereby a coating layer can be laminated on at least a part of the surface of the base material to obtain a laminate. In addition, the film-shaped coating agent may be swollen or heated to be softened in advance, placed so as to cover so as to cover at least a part of the surface of the base material, and then cured in a state of being brought into pressure contact with adherends. According to these methods, since a laminate is obtained using a biodegradable coating agent, there is an advantage that a laminate capable of reducing an environmental load at the time of disposal can be easily produced. Note that the base material may be any material as long as the film-shaped coating agent containing the polymer compound can be bonded to the base material, and the base material may be similar to an adherend to be bonded with the film-shaped adhesive containing the polymer compound.

A mechanism by which the film-shaped coating agent is bonded to the surface of the base material is considered to be similar to a mechanism by which the film-shaped adhesive is bonded to an adhesion surface of the adherend. Therefore, as a specific method for producing a laminate using the film-shaped coating agent, a similar method to the method used when an adhesive body is obtained using the film-shaped adhesive can be adopted. Note that, when a softened film-shaped coating agent is cured while being brought into pressure contact with the surface of the base material, for example, the film-shaped coating agent may be pressed against the surface of the base material using a pressing body similar to a pressing body used when the solution-state coating agent with which a top surface of the base material is coated is pressed against the surface of the base material. Similarly to the film-shaped adhesive, the film-shaped coating agent not only can be expected to have high adhesive strength to a base material, but also can be peeled off after being bonded, and furthermore can be reused.

As the powdery adhesive or the powdery coating agent containing the polymer compound, a powder composition containing a polymer compound powder obtained by the present embodiment method is used. This powder composition may contain auxiliary components such as various addition residues as long as the powder composition contains the polymer compound as a main component. As described above, in the polymer compound production method according to the present embodiment, a target polymer compound is obtained in a state of being dissolved in dimethyl sulfoxide (as a dimethyl sulfoxide solution). Therefore, a method for producing the powdery adhesive or the powdery coating agent according to the present embodiment includes a step of powderizing, by a known method, a polymer compound extracted from dimethyl sulfoxide by, for example, a known extraction method or separation method.

The polymer compound contained in the powdery adhesive or the powdery coating agent is not particularly limited as long as the polymer compound is obtained by the production method according to the present embodiment. For example, any of polymer compounds contained in a solution-state adhesive or a solution-state coating agent, a water-dispersible adhesive or a water-dispersible coating agent, and a film-shaped adhesive or a film-shaped coating agent can be used. Note that, as described above, the polymer compound according to the present embodiment exhibits higher affinity for an aqueous medium (aqueous liquid) containing water than a protein. Therefore, in such a powder adhesive or powdery coating agent containing the polymer compound, the content (dispersion amount) of a protein can be increased and the polymer compound can be uniformly dispersed in an aqueous medium as compared with a water-dispersible adhesive or a water-dispersible coating agent in which a protein is simply dispersed in an aqueous medium. Therefore, when a water-dispersible adhesive or a water-dispersible coating agent in which the polymer compound is dispersed in an aqueous medium is used, it can be expected that adhesive strength of the adhesive or the coating agent to an adhesion surface of an adherend or a laminate surface described later is improved as compared with a case of using a water-dispersible adhesive or a water-dispersible coating agent in which a protein is dispersed in an aqueous medium.

The powdery adhesive containing the modified protein is also used for producing an adhesive body in which a plurality of adherends is bonded to each other. For example, in a state where the powdery adhesive is interposed between a plurality of adherends to be bonded, the powdery adhesive is heated and pressurized via the adherends to solidify the powdery adhesive, whereby the adherends can be bonded to each other to obtain an adhesive body. According to such a method, since an adhesive body is obtained using a biodegradable adhesive, there is an advantage that an adhesive body capable of reducing an environmental load at the time of disposal can be easily produced. Note that the adherend may be any adherend as long as the adherend can be bonded with the powdery adhesive containing the polymer compound, and for example, the adherend may be similar to an adherend to be bonded with the solution-state adhesive containing the polymer compound.

A specific method for producing the adhesive body using the powdery adhesive is not also limited at all. For example, when the powdery adhesive interposed between a plurality of adherends to be bonded is heated and pressurized, the adherends are sandwiched by metal plates and the like from both sides opposite to the adhesion surface side, and the metal plates are heated to heat the powdery adhesive interposed between the adherends together with the adherends. At the same time, the metal plates are pressurized with a hand press machine or the like, and the powdery adhesive is pressurized for a predetermined time via the adherends. As a result, the powdery adhesive is converted into a resin and solidified to bond the adherends. Note that a heating temperature, a pressurization amount, and a heating and pressurization time when the powdery adhesive is solidified are appropriately selected from a range in which the modified protein can be converted into a resin according to the type of modified protein contained in the powdery adhesive.

The powdery coating agent containing the polymer compound is used for producing a laminate in which a coating layer is laminated on the whole or a part of a surface of a base material. For example, in a state where the powdery coating agent is placed on at least a part of a surface of a base material, the powdery coating agent is heated and pressurized between a pressurizing body and the base material to be solidified, whereby a coating layer is laminated on at least a part of the surface of the base material.

A specific method for producing a laminate using the powdery coating agent is not also limited at all. For example, a metal plate or the like is disposed so as to cover the entire powdery adhesive placed on the surface of the base material, and the metal plate is heated to heat the powdery adhesive. At the same time, the powdery adhesive is pressurized between the metal plate and the base material for a predetermined time with a hand press machine or the like, whereby the powdery coating agent is converted into a resin and solidified. Note that a heating temperature, a pressurization amount, and a heating and pressurization time of the powdery coating agent are similar to those when an adhesive body is obtained using the powdery adhesive.

Although some embodiments have been described above, the present disclosure is not limited to the above embodiments at all. The description contents of the above-described embodiments can be applied to each other.

### Examples

Hereinafter, the present disclosure will be described more specifically based on Examples and the like. Note that the present disclosure is not limited to the following Examples.

### [Preparation of polypeptide]

### (Preparation of expression vector)

An artificial fibroin having the amino acid sequence set forth in SEQ ID NO: 9 (PRT2882) was designed.

Note that an average hydropathy index value of the artificial fibroin having the amino acid sequence set forth in SEQ ID NO: 9 (PRT2882) is 0.47.

A nucleic acid encoding the artificial fibroin having the amino acid sequence set forth in SEQ ID NO: 9 was each synthesized. In the nucleic acid, an NdeI site was added to a 5' end and an EcoRI site was added downstream of a stop codon. The nucleic acid was cloned into a cloning vector (pUC118). Thereafter, the nucleic acid was subjected to restriction enzyme treatment with NdeI and EcoRI, and then, recombined into a polypeptide expression vector pET-22b(+), thereby obtaining an expression vector.

### (Preparation of polypeptide)

Escherichia coli BLR (DE3) was transformed with the obtained expression vector. The transformed Escherichia coli was cultured in 2 mL of an LB medium containing ampicillin for 15 hours. The culture solution was added to 100 mL of a seed culture medium (Table 2) containing ampicillin so that the OD₆₀₀ was 0.005. The culture solution was maintained at a temperature of 30°C, and placed in a culture flask (for about 15 hours) until OD₆₀₀ reached 5, thereby obtaining a seed culture solution.

**[Table 2]**

| Seed culture medium | | | |
|---|---|---|---|
| Reagent | | Concentration (g/L) | |
| | Glucose | | 5.0 |
| | KH₂PO₄ | | 4.0 |
| | K₂HPO₄ | | 9.3 |
| | Yeast Extract | | 6.0 |
| | Ampicillin | | 0.1 |

The seed culture solution was added to a jar fermenter containing 500 mL of a production medium (Table 3) so that the OD₆₀₀ was 0.05. The culture was carried out while keeping the culture solution temperature at 37°C and controlling the pH constant at 6.9. Furthermore, the culture solution was maintained to have a 20% dissolved oxygen concentration of the saturated dissolved oxygen concentration.

**[Table 3]**

| Production medium | | | |
|---|---|---|---|
| Reagent | | Concentration (g/L) | |
| | Glucose | | 12.0 |
| | KH₂PO₄ | | 9.0 |
| | MgSO₄·7H₂O | | 2.4 |
| | Yeast Extract | | 15 |
| | FeSO₄·7H₂O | | 0.04 |
| | MnSO₄·5H₂O | | 0.04 |
| | CaCl₂·2H₂O | | 0.04 |
| | Adekanol (ADEKA, LG-295S) | | 0.1 (mL/L) |

Immediately after glucose in the production medium was completely consumed, a feed solution (455 g/1 L of glucose, 120 g/1 L of Yeast Extract) was added to the production medium at a speed of 1 mL/min. The culture was carried out while keeping the culture solution temperature at 37°C and controlling the pH constant at 6.9. The culturing was performed for 20 hours while a dissolved oxygen concentration in the culture solution was maintained at 20% of the saturated dissolved oxygen concentration. The expression of the artificial fibroin was then induced by adding 1 M isopropyl-β-thiogalactopyranoside (IPTG) to the culture solution such that the final concentration was 1 mM. When 20 hours had elapsed after the addition of IPTG, the culture solution was centrifuged, and bacterial cells were collected. SDS-PAGE was conducted using the bacterial cells prepared from the culture solution obtained before the addition of IPTG and after the addition of IPTG. The expression of the target artificial fibroin that depended on the addition of IPTG was confirmed by emergence of a band having the size of the target artificial fibroin.

### (Purification of polypeptide)

Bacterial cells that were collected 20 hours after the addition of IPTG were washed with a 20 mM Tris-HCl buffer (pH 7.4). The washed bacterial cells were suspended in a 20 mM Tris-HCl buffer (pH 7.4) containing about 1 mM PMSF, and the cells were disrupted with a high-pressure homogenizer (manufactured by GEA Niro Soavi). The disrupted cells were centrifuged to obtain a precipitate. The obtained precipitate was washed with a 20 mM Tris-HCl buffer (pH 7.4) until the precipitate reached a high level of purity. The washed precipitate was suspended in an 8 M guanidine buffer (8 M guanidine hydrochloride, 10 mM sodium dihydrogen phosphate, 20 mM NaCl, 1 mM Tris-HCl, pH 7.0) so that the precipitate reached a concentration of 100 mg/mL, and then, the precipitate was dissolved by being stirred with a stirrer at 60°C for 30 minutes. After the precipitate was dissolved, the resulting solution was dialyzed with water using a dialysis tube (cellulose tube 36/32 manufactured by Sanko Junyaku Co., Ltd.).

A white aggregated polypeptide obtained after dialysis was collected by centrifugation, and moisture was removed with a lyophilizer and a lyophilized powder was collected, thereby obtaining a powdery artificial fibroin (PRT2882). It was confirmed that a disulfide bond was formed between molecules of an artificial fibroin having a cysteine residue inserted therein. It was confirmed that the artificial fibroin contained 13.8 parts by mass of a dimer, 2.1 parts by mass of a trimer, and 0.8 parts by mass of a tetramer with respect to 100 parts by mass of a monomer. Formation of a disulfide bond was measured by SDS-PAGE.

### [Preparation of compound having structure described in general formula (1)]

### (Synthesis of polyethylene glycol bismaleate)

500 g of polyethylene glycol (weight average molecular weight: 10,000) and 50 g of maleic anhydride were put in a container, and vacuum-nitrogen substitution was performed. After the contents were melted, 7 mL of triethylamine was added thereto, the temperature was raised to 80°C, and the mixture was heated and stirrer for 2.5 hours to cause the mixture to react. A precipitate was generated and collected by a reprecipitation method in which the solution after the reaction was added to 500 mL of cyclopentyl methyl ether (CPME). To the obtained precipitate, 1500 mL of CPME was added again, and the mixture was stirred to wash the precipitate. To the washed precipitate collected by filtration, 2000 mL of tetrahydrofuran was added, and the mixture was stirred to further wash the precipitate. Then, the precipitate was collected by filtration and vacuum-dried to obtain 450 g of polyethylene glycol bismaleate (polyethylene glycol having two maleic acid skeletons) (yield: 90% by mass).

### [Preparation of compound having structure described in general formula (2)]

### (Synthesis of polyethylene glycol bisacrylate)

300 mL of a solution in which 60 g of polyethylene glycol (weight average molecular weight: 10000) was dissolved in dichloromethane was prepared, and 60 g of anhydrous sodium sulfate was added thereto and the mixture was stirred for 30 minutes. The anhydrous sodium sulfate was collected by filtration, and the filter-collected product was washed with 100 mL of dichloromethane and a solid was removed to obtain a washing liquid. The obtained filtrate and washing liquid were put in a 1 L four-necked flask, and the solvent was distilled off under reduced pressure to obtain purified polyethylene glycol.

Next, under a nitrogen atmosphere, 300 mL of toluene previously dehydrated with molecular sieve 4A was added to the purified polyethylene glycol to prepare a suspension. The obtained suspension was heated to about 50°C under a nitrogen atmosphere to form a uniform solution. Toluene was distilled off from the obtained solution under reduced pressure conditions. To the generated residue, 300 mg of hydroquinone as a polymerization inhibitor was added, the mixture was dried under reduced pressure, and then a four-necked flask was filled with nitrogen gas.

Next, 500 mL of dichloromethane previously dehydrated with molecular sieve 4A was added into the four-necked flask using a cannula at room temperature and under a nitrogen stream. Furthermore, 8.4 mmL (60 mmol) of triethylamine previously dehydrated with molecular sieve 4A was added thereto using a syringe at room temperature and under a nitrogen atmosphere to prepare a mixed solution. The mixed solution was maintained under a nitrogen atmosphere and cooled using an ice bath adjusted to -15°C. While a reaction temperature was adjusted to -5°C to -3°C, 5 mL (60 mmol) of acrylic acid chloride was dropped for about five minutes under a nitrogen atmosphere. After dropping of acrylic acid chloride was completed, the temperature was naturally raised without removing the ice bath, and the mixture was stirred overnight under a nitrogen atmosphere to cause a reaction.

The solvent was distilled off from the solution after the reaction under reduced pressure, and the obtained residue was washed with n-hexane. To the washed residue, 600 mg of hydroquinone was added, and the mixture was subjected to thermal extraction using 700 mL of tetrahydrofuran to obtain an extract. The extract was filtered using Celite 503, and 700 mL of n-hexane was added to the filter-collected product to reprecipitate the filter-collected product. The generated precipitate was collected by filtration and dissolved in 500 mL of dichloromethane, 300 mg of hydroquinone was added thereto, and the solvent was distilled off under reduced pressure. The obtained residue was dried under reduced pressure to obtain 58.8 g (yield: 99% by mass of polyethylene glycol bisacrylate (polyether having two acrylate backbones) as a light yellow powder.

### [Preparation of other compounds]

### (Synthesis of polyethylene glycol bismaleimide)

As polyethylene glycol bismaleimide, Mal-PEG-Mal (Maleimide-PEG-Maleimide, molecular weight: 10000) manufactured by Funakoshi Co., Ltd. was used.

### (Example I-1)

To a two-necked eggplant flask, 1 g (0.1 mmol) of an artificial fibroin (polypeptide, 10 kDa) having the amino acid sequence set forth in SEQ ID NO: 9 (PRT2882) prepared as described above and 12.5 mg (0.08 mmol) of dithiothreitol (DTT) as a reducing agent were added, 12.5 g of dimethyl sulfoxide (DMSO) was added thereto, and the mixture was heated and stirred at 70°C for 30 minutes to prepare a solution A containing the polypeptide. Note that the blending amount of the reducing agent was adjusted to 0.8 equivalents with respect to a mercapto group of the polypeptide.

In a flask, 1 g (0.1 mmol) of polyethylene glycol bismaleate prepared as described above was weighed, 7.5 g of dimethyl sulfoxide (DMSO, manufactured by FUJIFILM Wako Pure Chemical Corporation) was added thereto, and the mixture was heated and stirred at 70°C for ten minutes for dissolution. In this way, a solution B containing polyethylene glycol bismaleate was prepared.

The solution A and the solution B were mixed, and 110 µL (0.08 mmol) of triethylamine (TEA, manufactured by FUJIFILM Wako Pure Chemical Corporation) was further added thereto as a base to prepare a reaction solution. Note that, in the mixed solution, the blending amount of the polypeptide was adjusted to 7 parts by mass with respect to 100 parts by mass of dimethyl sulfoxide. The blending amount of the base was adjusted to 8 parts by mass with respect to the total mass of the polypeptide. Next, the reaction solution was heated and stirred at 70°C for 90 minutes to allow the reaction to proceed. The solution after the reaction was centrifuged and a precipitate was removed, thereby obtaining a solution containing a polymer compound in which a polypeptide moiety and a polyethylene glycol moiety bind to each other. Note that the centrifugation was performed using a micro high-speed cooling centrifuge (manufactured by TOMY INDUSTRIAL CO., LTD., trade name: MX-307) under treatment conditions of 15000 rpm for ten minutes.

### <Evaluation of polymer compound production method: status of precipitate in solution after reaction>

The solution after the reaction before centrifugation was visually observed to confirm presence or absence of a precipitate. In addition, it was confirmed whether or not a gel-like substance was generated when the supernatant after removal of the precipitate was centrifuged under treatment conditions of 15000 rpm for ten minutes. From the obtained results, evaluation was performed according to the following criteria. Results thereof are presented in Table 4. Note that it was confirmed that the precipitate was mainly yellowish white and contained a byproduct generated by formation of a disulfide bond between peptides. In addition, the gel-like substance is a brown transparent solid.
A: No precipitate is observed, and no gel-like substance is generated.
B: A precipitate is observed, but no gel-like substance is generated.
C: No precipitate is observed, but a gel-like substance is generated.
D: A precipitate is observed, and a gel-like substance is also generated.

### <Evaluation of polymer compound production method: degree of conversion from raw material to target product>

The solution after the reaction prepared in Example I-1 was defoamed by centrifugation, and 3 g of the defoamed reaction solution was dropped onto a disposable tray (manufactured by AS ONE Corporation, trade name: balance dish SCC, outer dimension: 80 mm × 80 mm × 24 mm) and developed. Thereafter, the resulting solution was predried at 60°C for eight hours, and further dried at 80°C under reduced pressure for three hours to evaporate the solvent, thereby preparing a film having a film thickness of 0.09 mm. In a TFANa-HFIP solution (1.3 mL) in which 85.3 mg of sodium trifluoroacetate (TFANa, manufactured by FUJIFILM Wako Pure Chemical Corporation) was dissolved in 1000 g of HFIP, 2 mg of the obtained film and 3.1 mg of (tris(2-carboxyethyl) phosphine (TCEP) were dissolved to prepare a measurement sample. The measurement sample thus obtained was subjected to gel permeation chromatography measurement under conditions described later. In the obtained chromatogram, a peak intensity (peak height) of the polypeptide as a raw material was represented by P2, and a peak intensity (peak height) of the polymer compound as a product was represented by P1, and a ratio therebetween (P1/P2) was calculated. The higher this value is, the more the reaction proceeds. Results thereof are presented in Table 4.

Measurement conditions of the GPC measurement were as follows.
- Apparatus: Agilent 1260 Infinity II liquid chromatography system (Agilent Technologies, Inc.)
- Detector: Agilent 1260 Infinity II refractive index detector (RID) (Agilent Technologies, Inc.)
- Column: 0.5 µm guard column filter (Shodex GPC HK-G, Showa Denko K.K.), styrene-divinylbenzene copolymer column (Showdex GPC HK404L × 2, inner diameter: 4.6 mm× length: 150 mm, manufactured by Showa Denko K.K.)
- Eluent: TFANa-HFIP solution (a product in which 85.3 mg of sodium trifluoroacetate (TFANa) is dissolved in 1000 g of hexafluoroisopropanol (HFIP Central Glass Co., Ltd.))
- Flow rate: 0.15 mL/min
- Measurement temperature: 40°C

### (Example I-2)

A polymer compound in which a polypeptide moiety and a polyethylene glycol moiety bound to each other was obtained in a similar manner to Example I-1 except that components, blending amounts, and reaction temperature were prepared as presented in Table 4.

### (Example I-3)

To a two-necked eggplant flask, 1 g (0.1 mmol) of an artificial fibroin (polypeptide, 10 kDa) having the amino acid sequence set forth in SEQ ID NO: 9 (PRT2882) prepared as described above and 7.2 mg (0.08 mmol) of butanediol as a reducing agent were added, 9.0 g of dimethyl sulfoxide (DMSO) was added thereto, and the mixture was heated and stirred at 70°C for 30 minutes to prepare a solution C containing the polypeptide. Note that the blending amount of the reducing agent was adjusted to 0.8 equivalents with respect to a mercapto group of the polypeptide.

In a flask, 1 g (0.1 mmol) of polyethylene glycol bismaleate prepared as described above was weighed, 3.0 g of dimethyl sulfoxide (DMSO, manufactured by FUJIFILM Wako Pure Chemical Corporation) was added thereto, and the mixture was heated and stirred at 70°C for ten minutes for dissolution. In this way, a solution D containing polyethylene glycol bismaleate was prepared.

The solution C and the solution D were mixed, and 110 µL (0.08 mmol) of triethylamine (TEA, manufactured by FUJIFILM Wako Pure Chemical Corporation) was further added thereto as a base to prepare a reaction solution. Note that, in the mixed solution, the blending amount of the polypeptide was adjusted to 7 parts by mass with respect to 100 parts by mass of dimethyl sulfoxide. The blending amount of the base was adjusted to 8 parts by mass with respect to the total mass of the polypeptide. Next, the reaction solution was heated and stirred at 70°C for 90 minutes to allow the reaction to proceed. The solution after the reaction was centrifuged and a precipitate was removed, thereby obtaining a solution containing a polymer compound in which a polypeptide moiety and a polyethylene glycol moiety bind to each other. Note that the centrifugation was performed using a micro high-speed cooling centrifuge (manufactured by TOMY INDUSTRIAL CO., LTD., trade name: MX-307) under treatment conditions of 15000 rpm for ten minutes.

### (Example I-4)

A solution containing a polymer compound in which a polypeptide moiety and a polyethylene glycol moiety bound to each other was obtained in a similar manner to Example I-2 except that components, blending amounts, and reaction temperature were prepared as presented in Table 4.

### (Example I-5)

In a flask, 1 g (0.1 mmol) of polyethylene glycol bisacrylate prepared as described above and 25 mg (0.23 mmol) of hydroquinone (manufactured by FUJIFILM Wako Pure Chemical Corporation) as a polymerization inhibitor were weighed, 9.0 g of dimethyl sulfoxide (DMSO, manufactured by FUJIFILM Wako Pure Chemical Corporation) was added thereto, and the mixture was heated and stirred at 70°C for ten minutes for dissolution. In this way, a solution E containing polyethylene glycol bisacrylate was prepared.

A solution A prepared in a similar manner to Example I-1 and the solution E were mixed, and 110 µL (0.08 mmol) of triethylamine (TEA, manufactured by FUJIFILM Wako Pure Chemical Corporation) was further added thereto as a base to prepare a reaction solution. Note that, in the mixed solution, the polypeptide was adjusted to 7 parts by mass with respect to 100 parts by mass of dimethyl sulfoxide. Next, the reaction solution was heated and stirred at 70°C for 90 minutes to allow the reaction to proceed. The solution after the reaction was centrifuged and a precipitate was removed, thereby obtaining a solution containing a polymer compound in which a polypeptide moiety and a polyethylene glycol moiety bind to each other. Note that the centrifugation was performed using a micro high-speed cooling centrifuge (manufactured by TOMY INDUSTRIAL CO., LTD., trade name: MX-307) under treatment conditions of 15000 rpm for ten minutes.

### (Examples I-6 and I-7)

A solution containing a polymer compound in which a polypeptide moiety and a polyethylene glycol moiety bound to each other was obtained in a similar manner to Example I-5 except that components, blending amounts, and reaction temperature were prepared as presented in Table 4.

The obtained solutions after the reaction in Examples I-2 to I-7 were evaluated in a similar manner to Example I-1. Results thereof are presented in Table 4.

**[Table 4]**

| | | Example I-1 | Example I-2 | Example I-3 | Example I-4 | Example I-5 | Example I-6 | Example I-7 |
|---|---|---|---|---|---|---|---|---|
| (A) Polypeptide [mmol] | | 0.1 | 0.1 | 0.1 | 0.1 | 0.1 | 0.1 | 0.1 |
| (B) Compound | Polyethylene glycol bismaleate [mmol] | 0.1 | 0.1 | 0.1 | 0.1 | - | - | - |
| | Polyethylene glycol bisacrylate [mmol] | - | - | - | - | 0.1 | 0.1 | 0.1 |
| Other compound | Polyethylene glycol bismaleimide [mmol] | - | - | - | - | - | - | - |
| (C) Base | Triethylamine [mmol] | 0.08 | 0.08 | 0.08 | 0.08 | 0.16 | 0.16 | 0.16 |
| (D) Reducing agent | Dithiothreitol [mmol] | 0.08 | 0.08 | - | - | - | - | - |
| | Butanedithiol [mmol] | - | - | 0.08 | - | 0.08 | 0.16 | 0.24 |
| | Sodium sulfate [mmol] | - | - | - | 0.08 | - | - | - |
| (E) Polymerization inhibitor | Hydroquinone [mmol] | - | - | - | - | 0.23 | 0.23 | 0.23 |
| Solvent | Dimethyl sulfoxide [g] | 20 | 20 | 12 | 12 | 12 | 12 | 12 |
| Reaction temperature [°C] | | 70 | 60 | 70 | 70 | 70 | 70 | 70 |
| Evaluation | Peak intensity ratio in GPC: value of ([peak intensity P1 of product]/ [peak intensity P2 of unreacted polypeptide]) | 8.1 | 6.8 | 6.6 | 11.5 | 6.3 | 8.3 | 4.7 |
| | Presence or absence of precipitate in reaction system | A | A | A | B | B | A | B |

In Table 4, the amount of dimethyl sulfoxide means the total amount (for example, the sum of the amount of DMSO in the solution A and the amount of DMSO in the solution B) in the solution after mixing.

### (Comparative Example I-1)

In a flask, 1 g (0.1 mmol) of polyethylene glycol bismaleimide (Mal-PEG-Mal (Maleimide-PEG-Maleimide, molecular weight: 10000, manufactured by Funakoshi Co., Ltd.) was weighed, 12.5 g of dimethyl sulfoxide (DMSO, manufactured by FUJIFILM Wako Pure Chemical Corporation) was added thereto, and the mixture was heated and stirred at 70°C for ten minutes for dissolution. In this way, a solution F containing polyethylene glycol bismaleimide was prepared.

A solution A prepared in a similar manner to Example I-1 and the solution F were mixed, and 110 µL (0.08 mmol) of triethylamine (TEA, manufactured by FUJIFILM Wako Pure Chemical Corporation) was further added thereto as a base to prepare a reaction solution. Note that, in the mixed solution, the blending amount of the polypeptide was adjusted to 7 parts by mass with respect to 100 parts by mass of dimethyl sulfoxide. Next, the reaction solution was heated and stirred at 70°C for 90 minutes to allow the reaction to proceed. The solution after the reaction was centrifuged to obtain a polymer compound in which a polypeptide moiety and a polyethylene glycol moiety bound to each other as a precipitate. Note that the centrifugation was performed using a micro high-speed cooling centrifuge (manufactured by TOMY INDUSTRIAL CO., LTD., trade name: MX-307) under treatment conditions of 15000 rpm for ten minutes.

### (Comparative Example I-2)

To a two-necked eggplant flask, 1 g (0.1 mmol) of an artificial fibroin (polypeptide, 10 kDa) having the amino acid sequence set forth in SEQ ID NO: 9 (PRT2882) prepared as described above and 12.5 mg (0.08 mmol) of dithiothreitol (DTT) as a reducing agent were added, 12.5 g of dimethyl sulfoxide (DMSO) was added thereto, and the mixture was heated and stirred at 70°C for 30 minutes to prepare a solution G containing the polypeptide. Note that the blending amount of the reducing agent was adjusted to 0.8 equivalents with respect to a mercapto group of the polypeptide.

In a flask, 1 g (0.1 mmol) of polyethylene glycol bismaleimide (Mal-PEG-Mal (Maleimide-PEG-Maleimide, molecular weight: 10000, manufactured by Funakoshi Co., Ltd.) was weighed, 25.0 g of dimethyl sulfoxide (DMSO, manufactured by FUJIFILM Wako Pure Chemical Corporation) was added thereto, and the mixture was heated and stirred at 70°C for ten minutes for dissolution. In this way, a solution H containing polyethylene glycol bismaleimide was prepared.

The solution G and the solution H were mixed, and 110 µL (0.08 mmol) of triethylamine (TEA, manufactured by FUJIFILM Wako Pure Chemical Corporation) was further added thereto as a base to prepare a reaction solution. Note that, in the mixed solution, the blending amount of the polypeptide was adjusted to 7 parts by mass with respect to 100 parts by mass of dimethyl sulfoxide. Next, the reaction solution was heated and stirred at 70°C for 90 minutes to allow the reaction to proceed. The solution after the reaction was centrifuged to obtain a polymer compound in which a polypeptide moiety and a polyethylene glycol moiety bound to each other as a precipitate. Note that the centrifugation was performed using a micro high-speed cooling centrifuge (manufactured by TOMY INDUSTRIAL CO., LTD., trade name: MX-307) under treatment conditions of 15000 rpm for ten minutes.

The obtained solutions after the reaction in Comparative Examples I-1 and I-2 were evaluated in a similar manner to Example I-1. Results thereof are presented in Table 5. In Table 5, regarding Comparative Example 1, the peak intensity ratio in GPC is "-", which indicates that the measurement itself could not be performed due to precipitation and a gel-like substance in the reaction system.

**[Table 5]**

| | | Comparative Example I-1 | Comparative Example I-2 |
|---|---|---|---|
| (A) Polypeptide [mmol] | | 0.1 | 0.1 |
| (B) Compound | Polyethylene glycol bismaleate [mmol] | - | - |
| | Polyethylene glycol bisacrylate [mmol] | - | - |
| Other compound | Polyethylene glycol bismaleimide [mmol] | 0.1 | 0.1 |
| (C) Base | Triethylamine [mmol] | 0.08 | 0.08 |
| (D) Reducing agent | Dithiothreitol [mmol] | 0.08 | 0.08 |
| | Butanedithiol [mmol] | - | - |
| | Sodium sulfate [mmol] | - | - |
| (E) Polymerization inhibitor | Hydroquinone [mmol] | - | - |
| Solvent | Dimethyl sulfoxide [g] | 20 | 40 |
| Reaction temperature [°C] | | 70 | 70 |
| Evaluation | Peak intensity ratio in GPC: value of ([peak intensity P1 of product]/ [peak intensity P2 of unreacted polypeptide]) | - | 3.2 |
| | Presence or absence of precipitate in reaction system | D | B |

### (Example I-8)

The reaction solution prepared in Example I-3 was defoamed by centrifugation, and 3 g of the defoamed reaction solution was dropped onto a disposable tray (AS ONE Corporation, trade name: balance dish SCC, outer dimension: 80 mm × 80 mm × 24 mm) and developed. Thereafter, the resulting solution was dried at 60°C for eight hours, and further dried at 80°C under reduced pressure for three hours to evaporate the solvent, thereby preparing a film having a film thickness of 0.09 mm.

### <Evaluation of film>

Breaking elongation, elastic modulus, and toughness of the film prepared as described above were measured using a tensile tester (trade name: AG-X plus50kN manufactured by Shimadzu Corporation). The film was used after being stored for 24 hours or more under an environment of 20°C and relative humidity: 65%. After the storage, the film was punched into a dumbbell-shaped test piece using a punching machine (manufactured by Tester Sangyo Co., Ltd., trade name: SA-1008) such that a film distance at a measurement portion was 25 mm and a width thereof was 10 mm to prepare a test piece. The test piece was set in the tensile tester, and a tensile test was performed under conditions of a load cell: 50 kN, a distance between grips: 25 mm, and a tensile speed: 10 mm/min. Results thereof are presented in Table 6.

### (Example I-9)

A film was prepared in a similar manner to Example I-8 except that the polymer material was changed to that prepared in Example I-7. The obtained film was evaluated in a similar manner to Example I-8. Results thereof are presented in Table 6.

### (Comparative Example I-3)

A film was prepared in a similar manner to Example I-8 except that the polymer material was changed to that prepared in Comparative Example I-2. The obtained film was evaluated in a similar manner to Example I-8. Results thereof are presented in Table 6.

**[Table 6]**

| | Example I-8 | Example I-9 | Comparative Example I-3 |
|---|---|---|---|
| Polymer compound | Polymer compound of Example I-3 | Polymer compound of Example I-7 | Polymer compound of Comparative Example I-2 |
| Breaking elongation [%] | 449 | 532 | 118 |
| Value of breaking strength [MPa]/yield point strength [MPa] | 1.42 | 1.63 | 1.37 |

As presented in Table 6 above, it was confirmed that since a side reaction such as disulfide bond formation between polypeptides was suppressed, no precipitate was generated, and a film capable of exhibiting favorable physical properties was obtained even though each of the polymer-containing solutions prepared in Examples I-8 and I-9 was used as it without purification.

### (Example I-10)

To 100 g of ethyl acetate prepared in a 300 mL beaker, 100 g of a solution prepared in a similar manner to Example I-1 was gradually dropped under stirring A precipitate generated in the beaker was finely pulverized using a homogenizer (manufactured by IKA, trade name: T-18 Digital). Thereafter, the precipitate was collected by suction filtration. The filter-collected product was put in 100 g of ethyl acetate, the mixture was stirred, and the filter-collected product was washed. Washing with ethyl acetate was repeated two times, and then collection by filtration was performed and vacuum-drying was performed at 40°C for two hours to obtain a purified polymer compound powder.

To a 20 mL glass vial (AS ONE, trade name: screw vial bottle) containing a stirrer, 6 g of the obtained powder was added, and then 11.46 mL of formic acid (manufactured by Nichiwa Gousei Co., Ltd., concentration: 98% by mass) was added thereto. Thereafter, the mixture was stirred at 45°C for two hours to prepare a formic acid solution (dope solution) having a concentration of 30% by mass. A viscosity of the obtained dope solution at a temperature of 45°C was 17300 mPa•s. Here, for the viscosity of the dope solution, the dope solution was filled into a glass tube containing a spherical probe (φ4.7 mm). The viscosity is a value measured at a measurement temperature of 45°C using an EMS viscometer (manufactured by Kyoto Electronics Manufacturing Co., Ltd., trade name: EMS-1000S).

Into a 50 mL disposable syringe (manufactured by Musashi Engineering Co., Ltd., trade name: PSY-50E), 10.0 mL of the dope solution was filled, and then a plunger (manufactured by Musashi Engineering Co., Ltd., trade name: FLP-50E) was inserted thereinto. Then, a plastic needle (manufactured by Musashi Engineering Co., Ltd., gauge: 22, inner diameter: 0.47 mm) was attached thereto, and an adapter tube (manufactured by Musashi Engineering Co., Ltd., trade name: AT-50E-H-1.0) was attached to an upper part of the syringe. In addition, a syringe heater was attached thereto, and the dope solution was maintained at 45°C.

Thereafter, a syringe pump maintained at 45°C was operated while nitrogen of 0.2 MPa was caused to flow therein, whereby the dope solution was discharged at a linear velocity of 1.00 m/min. The discharged dope solution was caused to pass through a drying line at 200°C and then caused to pass through a take-up roller adjusted to a rotation speed of 10 m/min.

Thereafter, the dope solution was caused to pass through a drying line at 275°C via two rollers and wound up by a winding machine at a rotation speed of 15 m/min to obtain a fiber (filament). The mass per 1 m of the wound fiber was measured, and the mass per 10000 m (fineness, unit: dtex) was calculated. A sample obtained by bundling five filaments described above was prepared, and cross-sectional analysis was performed using a polarizing microscope (manufactured by Nikon Corporation, trade name: ECLIPSE LV100ND) to determine equivalent circle diameters of the five filaments, and an arithmetic average thereof was adopted as an equivalent circle diameter of the filaments.

### <Evaluation of fiber: measurement of breaking elongation and breaking strength>

The sample obtained by bundling the five filaments was evaluated for breaking elongation and breaking strength using a tensile tester (manufactured by Shimadzu Corporation, AG-X plus50kN). The sample was stored for 24 hours or more under an environment of 20°C and relative humidity: 65% before use. The sample was set in the tester, and stretched until the sample was cut under conditions of a load cell: 50 kN, a distance between grips: 50 mm, and a tensile speed: 50 cm/min. The stress and the length at break were measured three times, and an arithmetic average value thereof was calculated. Results thereof are presented in Table 7.

### <Evaluation of fiber: measurement of stress relaxation>

The sample obtained by bundling the five filaments was evaluated for stress relaxation using a tensile tester (manufactured by Shimadzu Corporation, trade name: AG-X plus50kN). The sample was stored for 24 hours or more under an environment of 20°C and relative humidity: 65% before use. The sample set in the tensile tester, and a tensile test was performed under conditions of a load cell: 50 kN, a distance between grips: 50 mm, and a tensile speed: 50 cm/min. At this time, a stress X when the sample of 50 mm was stretched by 300% was measured. Thereafter, the sample was held in this state for 30 seconds, and a stress Y immediately after the holding was measured. This measurement was performed three times, and calculation was performed by a formula (X-Y)/X × 100, and then an arithmetic average thereof was defined as stress relaxation. Results thereof are presented in Table 7.

### (Reference Example)

A commercially available urethane fiber (manufactured by TORAY OPELONTEX CO., LTD., trade name: LYCRA T-127C) was evaluated in a similar manner to Example I-10. Results thereof are presented in Table 7.

**[Table 7]**

| | | Example I-10 | Reference Example |
|---|---|---|---|
| Polymer material | | Polymer material prepared in Example I-1 | Urethane fiber |
| Viscosity of dope solution at 45°C [mPa•s] | | 17300 | - |
| Evaluation | Fineness [dtex] | 30 | 22 |
| | Equivalent circle diameter [um] | 51 | 37 |
| | Value of breaking strength [MPa]/ yield point strength [MPa] | 1.53 | 8.94 |
| | Stress relaxation [%] | 46 | 34 |

### (Example II-1)

To 3.13 g of a solution containing a polymer compound in which a polypeptide moiety and a polyethylene glycol moiety bound to each other, which was prepared in a similar manner to Example I-1, 50 mg of a water-soluble thermoplastic resin (manufactured by Meisei Chemical Industry Co., Ltd., trade name: ALCOX E-75) was added, and the mixture was stirred at 70°C for one hour to prepare a DMSO solution (dope solution). A viscosity of the obtained dope solution at 25°C was 4130 mPa•s. Here, the viscosity of the dope solution is a value measured at a measurement temperature of 25°C using a digital viscometer (manufactured by Ametec Corporation, trade name: Brookfield DV2TLVTJ0).

The dope solution was transferred into a 100 mL glass beaker. Then, the dope was slowly stirred using a stainless steel spatula such that air bubbles did not enter the dope solution, and slowly lifted to stretch the dope solution into a fibrous form using consistency. The stretched fibrous dope solution was allowed to stand on a release film, and vacuum-dried for 15 hours using a vacuum oven at 80°C to obtain a filament (a polymer compound molded into a fibrous form).

A sample obtained by bundling five filaments described above was prepared, and cross-sectional analysis was performed using a polarizing microscope (manufactured by Nikon Corporation, trade name: ECLIPSE LV100ND) to determine equivalent circle diameters of the five filaments, and an arithmetic average thereof was adopted as an equivalent circle diameter of the filaments.

The resulting sample obtained by bundling the five filaments was evaluated in a similar manner to Example I-10. Results thereof are presented in Table 8. In Table 8, "-" indicates that measurement was not performed.

**[Table 8]**

| | | Example II-1 | Reference Example |
|---|---|---|---|
| Polymer material | | Polymer material prepared in Example I-1 | Urethane fiber |
| Viscosity of dope solution at 25°C [mPa•s] | | 4130 | - |
| Evaluation | Fineness [dtex] | 54 | 22 |
| | Equivalent circle diameter [µm] | 73 | 37 |
| | Value of breaking strength [MPa]/ yield point strength [MPa] | 4.50 | 8.94 |
| | Stress relaxation [%] | 44 | 34 |

### (Comparative Example I-4)

An attempt was made to prepare a fiber by changing the polymer material to that prepared in Comparative Example I-2, but the concentration of the solution was insufficient, and a fiber could not be prepared.

### (Examples III-1 and III-4)

A solution containing a polymer compound in which a polypeptide moiety and a polyethylene glycol moiety bound to each other was obtained in a similar manner to Example I-1 except that components, blending amounts, and reaction temperature were prepared as presented in Table 9.

The obtained solutions after the reaction in Examples III-1 and III-4 were evaluated in a similar manner to Example I-1. Results thereof are presented in Table 9.

**[Table 9]**

| | | Example III-1 | Example III-2 | Example III-3 | Example III-4 |
|---|---|---|---|---|---|
| (A) Polypeptide [mmol] | | 0.1 | 0.1 | 0.1 | 0.1 |
| (B) Compound | Polyethylene glycol bismaleate [mmol] | 0.1 | 0.1 | 0.1 | 0.1 |
| | Polyethylene glycol bisacrylate [mmol] | - | - | - | - |
| Other compound | Polyethylene glycol bismaleimide [mmol] | - | - | - | - |
| (C) Base | Triethylamine [mmol] | - | 0.08 | 0.16 | 0.32 |
| (D) Reducing agent | Dithiothreitol [mmol] | 0.08 | 0.08 | 0.08 | 0.08 |
| | Butanedithiol [mmol] | - | - | - | - |
| | Sodium sulfate [mmol] | - | - | - | - |
| (E) Polymerization | Hydroquinone [mmol] | - | - | - | - |
| inhibitor | | | | | |
| Solvent | Dimethyl sulfoxide [g] | 12 | 12 | 12 | 12 |
| Reaction temperature [°C] | | 70 | 70 | 70 | 70 |
| Evaluation | Peak intensity ratio in GPC: value of ([peak intensity P1 of product]/ [peak intensity P2 of unreacted polypeptide]) | 8.06 | 12.5 | 8.22 | 8.53 |
| | Presence or absence of precipitate in reaction system | A | A | C | C |

### (Examples III-5 and III-6)

A solution containing a polymer compound in which a polypeptide moiety and a polyethylene glycol moiety bound to each other was obtained in a similar manner to Example I-1 except that components, blending amounts, and reaction temperature were prepared as presented in Table 10.

The obtained solutions after the reaction in Examples III-5 and III-6 were evaluated in a similar manner to Example I-1. Results thereof are presented in Table 10.

### (Comparative Example III-1 and Examples III-7 and III-8)

A solution containing a polymer compound in which a polypeptide moiety and a polyethylene glycol moiety bound to each other was obtained in a similar manner to Example I-1 except that components, blending amounts, and reaction temperature were prepared as presented in Table 10.

The obtained solutions after the reaction in Comparative Example III-1 and Examples III-7 and III-8 were evaluated in a similar manner to Example I-1. Results thereof are presented in Table 10.

**[Table 10]**

| | | Example III-5 | Example III-6 | Comparative Example III-1 | Example III-7 | Example III-8 |
|---|---|---|---|---|---|---|
| (A) Polypeptide [mmol] | | 0.1 | 0.1 | 0.1 | 0.1 | 0.1 |
| (B) Compound | Polyethylene glycol bismaleate [mmol] | 0.1 | 0.1 | 0.1 | 0.1 | 0.1 |
| | Polyethylene glycol bisacrylate [mmol] | - | - | - | - | - |
| Other compound | Polyethylene glycol bismaleimide [mmol] | - | - | - | - | - |
| (C) Base | Triethylamine [mmol] | - | - | 0.08 | 0.08 | 0.08 |
| | Sodium carbonate [mmol] | 0.08 | - | - | - | - |
| | Potassium carbonate [mmol] | - | 0.08 | - | - | - |
| (D) Reducing agent | Dithiothreitol [mmol] | 0.08 | 0.08 | - | 0.06 | 0.08 |
| | Butanedithiol [mmol] | - | - | - | - | - |
| | Sodium sulfate [mmol] | - | - | - | - | - |
| (E) | Hydroquinone | - | - | - | - | - |
| Polymerization inhibitor | [mmol] | | | | | |
| Solvent | Dimethyl sulfoxide [g] | 12 | 12 | 12 | 12 | 12 |
| Reaction temperature [°C] | | 70 | 70 | 70 | 70 | 70 |
| Evaluation | Peak intensity ratio in GPC: value of ([peak intensity P1 of product]/ [peak intensity P2 of unreacted polypeptide]) | 6.54 | 6.75 | 0.73 | 5.27 | 12.5 |
| | Presence or absence of precipitate in reaction system | A | A | B | A | A |

### (Examples III-9 to III-11)

A solution containing a polymer compound in which a polypeptide moiety and a polyethylene glycol moiety bound to each other was obtained in a similar manner to Example I-1 except that components, blending amounts, and reaction temperature were prepared as presented in Table 11.

The obtained solutions after the reaction in Examples III-9 to III-11 were evaluated in a similar manner to Example I-1. Results thereof are presented in Table 11. In Table 11, the results of Example I-1 and Example III-2 are also presented for reference.

**[Table 11]**

| | | Example I-1 | Example III-2 | Example III-9 | Example III-10 | Example III-11 |
|---|---|---|---|---|---|---|
| (A) Polypeptide [mmol] | | 0.1 | 0.1 | 0.1 | 0.1 | 0.1 |
| (B) Compound | Polyethylene glycol bismaleate [mmol] | 0.1 | 0.1 | 0.1 | 0.1 | 0.1 |
| | Polyethylene glycol bisacrylate [mmol] | - | - | - | - | - |
| Other compound | Polyethylene glycol bismaleimide [mmol] | - | - | - | - | - |
| (C) Base | Triethylamine [mmol] | 0.08 | 0.08 | 0.08 | 0.08 | 0.16 |
| (D) Reducing agent | Dithiothreitol [mmol] | 0.08 | 0.08 | 0.08 | 0.08 | 0.08 |
| | Butanedithiol [mmol] | - | - | - | - | - |
| | Sodium sulfate [mmol] | - | - | - | - | - |
| **(E)** Polymerization inhibitor | Hydroquinone [mmol] | - | - | - | - | - |
| Solvent | Dimethyl sulfoxide [g] | 20 | 12 | 10 | 9 | 6 |
| Reaction temperature [°C] | | 70 | 70 | 70 | 70 | 70 |
| Evaluation | Peak intensity ratio in GPC: value of ([peak intensity P1 of product]/ [peak intensity P2 of unreacted polypeptide]) | 8.1 | 12.5 | 9.27 | 12.1 | 4.88 |
| | Presence or absence of precipitate in reaction system | A | A | C | C | C |

### (Example I-11)

### <Production of film-shaped adhesive>

To a two-necked eggplant flask, 2.5 g (0.25 mmol) of an artificial fibroin (polypeptide, 10 kDa) having the amino acid sequence set forth in SEQ ID NO: 9 (PRT2882) prepared as described above and 31.2 mg (0.2 mmol) of dithiothreitol (DTT) as a reducing agent were added, 25 g of dimethyl sulfoxide (DMSO) was added thereto, and the mixture was heated and stirred at 70°C for 30 minutes to prepare a solution I containing the polypeptide. Note that the blending amount of the reducing agent was adjusted to 0.8 equivalents with respect to a mercapto group of the polypeptide.

In a flask, 2.5 g (0.025 mmol) of polyethylene glycol bismaleate prepared as described above was weighed, 20 g of dimethyl sulfoxide (DMSO, manufactured by FUJIFILM Wako Pure Chemical Corporation) was added thereto, and the mixture was heated and stirred at 70 for ten minutes for dissolution. In this way, a solution J containing polyethylene glycol bismaleate was prepared.

The solution I and the solution J were mixed, and 275 µL (0.2 mmol) of triethylamine (TEA, manufactured by FUJIFILM Wako Pure Chemical Corporation) was further added thereto as a base to prepare a reaction solution. Note that, in the mixed solution, the blending amount of the polypeptide was adjusted to 6 parts by mass with respect to 100 parts by mass of dimethyl sulfoxide. The blending amount of the base was adjusted to 8 parts by mass with respect to the total mass of the polypeptide. Next, the reaction solution was heated and stirred at 70°C for 90 minutes to allow the reaction to proceed. The solution after the reaction was centrifuged and defoamed, thereby obtaining a DMSO solution containing a polymer compound in which a polypeptide moiety and a polyethylene glycol moiety bound to each other. Note that the centrifugation was performed using a micro high-speed cooling centrifuge (manufactured by TOMY INDUSTRIAL CO., LTD., trade name: MX-307) under treatment conditions of 15000 rpm for ten minutes.

Subsequently, the obtained DMSO solution of the polymer compound was dropped onto a stainless steel plate provided with a PET sheet (Teijin Film Solution Co., Ltd., Purex, A54 type, thickness: 38 µm), and developed by a frame applicator with an analog micrometer (IMOTO MFG. CO., LTD., coating width: 250 mm, gap: 1 mm) and a coating machine (IMOTO MFG. CO., LTD., IMC-7370, coating speed: 10 mm/sec). Subsequently, the resulting solution was dried at 60°C for eight hours, and further dried at 80°C under reduced pressure for three hours to evaporate DMSO, thereby obtaining a film-shaped adhesive having a thickness of 0.08 mm.

### <Production of adhesive body>

The film-shaped adhesive obtained as described above was cut into 15 mm × 15 mm, and then the cut film-shaped adhesive was immersed in water for five minutes to be swollen and softened. Subsequently, the softened film-shaped adhesive was placed on a region of 15 mm × 15 mm at one end of a wooden plate (made of Japanese cypress, 1.5 mm × 40 mm × 2 mm), and another wooden plate of the same size was superimposed thereon such that the film-shaped adhesive was interposed between the two wooden plates. Thereafter, the two wooden plates were fixed with a clip such that the film-shaped adhesive was brought into pressure contact with the wooden plates, placed in a blown constant temperature thermostat, and dried at 60°C for one hour to cure the film-shaped adhesive. As a result, an adhesive body A in which the two wooden plates were bonded with a film-shaped adhesive was obtained. For comparison, an operation similar to that described above was performed except that a commercially available double-sided tape (Nichiban, 15 mm wide, Nicetack NW-15) was used in place of the film-shaped adhesive to obtain an adhesive body B in which two pieces of wood were bonded with the double-sided tape.

### <Tensile test of adhesive body>

Each of the adhesive body A and the adhesive body B obtained as described above was set in a tensile tester (AG-X plus50kN, manufactured by Shimadzu Corporation) under an environment of a room temperature of 20°C and a humidity of 65%, and was subjected to a tensile test. Note that the tensile test was performed under conditions of a load cell: 50 kN, a distance between grips: 57.5 mm, and a tensile speed: 10 mm/min. As a result, tensile strength of the adhesive body A was 10 times or more larger than tensile strength of the adhesive body B. As a result, it was found that the film-shaped adhesive had sufficiently large adhesive force (adhesive strength) as compared with a commercially available double-sided tape or the like.

### <Peeling test of adhesive body>

Another adhesive body A was produced in such a manner as described above, and the film-shaped adhesive protruding from a gap of an adhesive portion in the adhesive body A was drawn from the adhesive portion. As a result, the two wooden plates bonded to each other could be peeled off from each other. In addition, when surfaces of the two wooden plates bonded to each other were visually observed, no change was observed from the surfaces of the wooden plates before bonding. Furthermore, the drawn film-shaped adhesive was washed with water, and then an operation similar to that in production of the adhesive body A was performed using this film-shaped adhesive and two other wooden plates. As a result, the other two wooden plates could be also bonded to each other. This indicates that the film-shaped adhesive can be reused.

### (Example I-12)

### <Production of powdery adhesive>

In a three-neck separable flask, 319.3 g of dimethyl sulfoxide (DMSO) was weighed, and 419 mg (2.72 mmol) of dithiothreitol (DTT) as a reducing agent was added thereto, and the mixture was stirred for dissolution. Thereafter, 27.5 g (2.75 mmol) of an artificial fibroin (polypeptide, 10 kDa) having the amino acid sequence set forth in SEQ ID NO: 9 (PRT2882) prepared as described above was added thereto, and the mixture was heated and stirred at 70°C for 30 minutes to prepare a solution K containing the polypeptide. Note that the blending amount of the reducing agent was adjusted to about 1 equivalent with respect to a mercapto group of the polypeptide.

In a flask, 27.5 g (2.75 mmol) of polyethylene glycol bismaleate prepared as described above was weighed, 125.5 g of dimethyl sulfoxide (DMSO, manufactured by FUJIFILM Wako Pure Chemical Corporation) was added thereto, and the mixture was heated and stirred at 70 for ten minutes for dissolution. In this way, a solution L containing polyethylene glycol bismaleate was prepared.

The solution K and the solution L were mixed, and 4400 mg (43.5 mmol) of triethylamine (TEA, manufactured by FUJIFILM Wako Pure Chemical Corporation) was further added thereto as a base to prepare a reaction solution. Note that, in the mixed solution, the blending amount of the polypeptide was adjusted to 5.5 parts by mass with respect to 100 parts by mass of dimethyl sulfoxide. The blending amount of the base was adjusted to 16 parts by mass with respect to the total mass of the polypeptide. Next, the reaction solution was heated and stirred at 70°C for 90 minutes to allow the reaction to proceed. The solution after the reaction was centrifuged and a precipitate was removed, thereby obtaining a DMSO solution containing a polymer compound in which a polypeptide moiety and a polyethylene glycol moiety bound to each other. Note that the centrifugation was performed under treatment conditions of 15000 rpm for 10 minutes.

The obtained DMSO solution of the polymer compound was repeatedly washed and centrifuged in acetone five times, and then the supernatant was removed by decantation to separate a precipitate. The obtained precipitate was spread on a tray and dried in a draft for several hours, and then dried in a vacuum oven at 40°C overnight to obtain a polymer compound powder. The residual DMSO of this powder was 3% by mass or less.

The obtained polymer compound powder was used as a powdery adhesive.

### <Production of laminate>

0.5 g of the powdery adhesive was weighed and placed on a part of one surface of a cotton woven fabric as a base material. Thereafter, the base material on which the powdery adhesive was placed was sandwiched between two stainless steel plates coated with a release agent, and then pressurized at 10 MPa for three minutes with a hand press machine while being heated at a temperature of 100°C. As a result, the powdery adhesive was resinified and solidified. As a result, a laminate in which a coating layer was laminated on a part of a base material surface was obtained.

### Industrial Applicability

According to the present disclosure, it is possible to provide a production method capable of increasing a raw material conversion ratio as compared with a conventional method in the above-described production of a polymer compound.

## Claims

1. A polymer compound production method comprising subjecting
a polypeptide having at least one mercapto group, and
at least one compound selected from the group consisting of a polyether, a polyester, and a polycarbonate and having two structures represented by the following general formula (1): 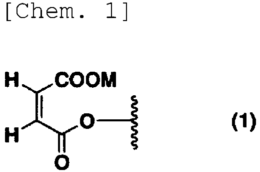
[In general formula (1), M represents any one of H, Na, K, NHEt₃, and NHEtiPr₂]
to a heating reaction in dimethyl sulfoxide in presence of a base and a reducing agent.

2. A polymer compound production method comprising subjecting
a polypeptide having at least one mercapto group, and
at least one compound selected from the group consisting of a polyether, a polyester, and a polycarbonate and having two structures represented by the following general formula (2): 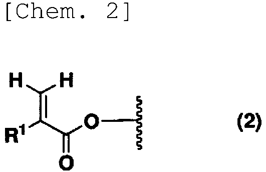
[In general formula (2), R¹ represents a hydrogen atom or a methyl group]
to a heating reaction in dimethyl sulfoxide in presence of a base, a reducing agent, and a polymerization inhibitor.

3. A polymer compound production method comprising subjecting
a polypeptide having at least one mercapto group, and
at least one compound selected from the group consisting of a polyether, a polyester, and a polycarbonate and having two structures represented by the following general formula (1): 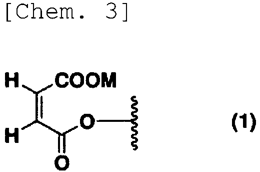
[In general formula (1), M represents any one of H, Na, K, NHEt₃, and NHEtiPr₂]
to a heating reaction in dimethyl sulfoxide in presence of a reducing agent.

4. A polymer compound production method comprising subjecting
a polypeptide having at least one mercapto group, and
at least one compound selected from the group consisting of a polyether, a polyester, and a polycarbonate and having two structures represented by the following general formula (2): 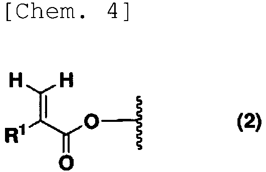
[In general formula (2), R¹ represents a hydrogen atom or a methyl group]
to a heating reaction in dimethyl sulfoxide in presence of a reducing agent and a polymerization inhibitor.

5. The polymer compound production method according to any one of claims 1 to 4, wherein the reducing agent is at least one selected from the group consisting of a dithiol, sodium sulfate, sodium sulfite, and sodium dithionite.

6. The production method according to any one of claims 1 to 4, wherein a blending amount of the reducing agent is 0.5 equivalents or more with respect to a mercapto group of the polypeptide.

7. The production method according to any one of claims 1 to 4, wherein the reaction between the polypeptide and the compound is performed at 50°C or higher.

8. A method for producing a polymer compound solution, the method comprising dissolving a polymer compound obtained by the production method according to any one of claims 1 to 4 in a solvent.

9. A method for producing a film, the method comprising molding a film from a polymer compound solution obtained by the production method according to claim 8.

10. A method for producing a fiber, the method comprising spinning a polymer compound solution obtained by the production method according to claim 8.

11. A method for producing a film, the method comprising:
subjecting a polypeptide having at least one mercapto group and at least one compound selected from the group consisting of a polyether, a polyester, and a polycarbonate and having two structures represented by the following general formula (1) to a heating reaction in dimethyl sulfoxide in presence of a base and a reducing agent to obtain a polymer compound solution; and
molding a film from the polymer compound solution. 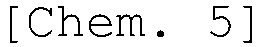 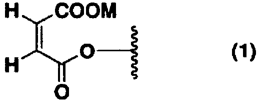
[In general formula (1), M represents any one of H, Na, K, NHEt₃, and NHEtiPr₂]

12. A method for producing a film, the method comprising:
subjecting a polypeptide having at least one mercapto group and at least one compound selected from the group consisting of a polyether, a polyester, and a polycarbonate and having two structures represented by the following general formula (2) to a heating reaction in dimethyl sulfoxide in presence of a base, a reducing agent, and a polymerization inhibitor; and
molding a film from the polymer compound solution. 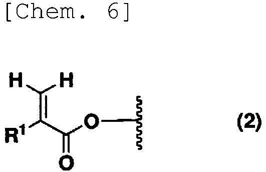
[In general formula (2), R¹ represents a hydrogen atom or a methyl group]

13. A method for producing a fiber, the method comprising:
subjecting a polypeptide having at least one mercapto group and at least one compound selected from the group consisting of a polyether, a polyester, and a polycarbonate and having two structures represented by the following general formula (1) to a heating reaction in dimethyl sulfoxide in presence of a base and a reducing agent to obtain a polymer compound solution; and
spinning the polymer compound solution. 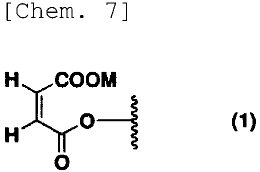
[In general formula (1), M represents any one of H, Na, K, NHEt₃, and NHEtiPr₂]

14. A method for producing a fiber, the method comprising:
subjecting a polypeptide having at least one mercapto group and at least one compound selected from the group consisting of a polyether, a polyester, and a polycarbonate and having two structures represented by the following general formula (2) to a heating reaction in dimethyl sulfoxide in presence of a base, a reducing agent, and a polymerization inhibitor; and
spinning the polymer compound solution. 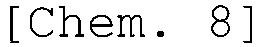 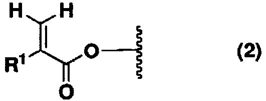
[In general formula (2), R¹ represents a hydrogen atom or a methyl group]

15. A film comprising a polymer compound, wherein
the polymer compound has
a structure in which a polypeptide moiety and
at least one structural unit selected from the group consisting of a polyether structure, a polyester structure, and a polycarbonate structure directly bind to each other by a structure represented by the following general formula (3), (4), or (5) such that the polypeptide moiety binds to S of the following general formula (3), (4), or (5), 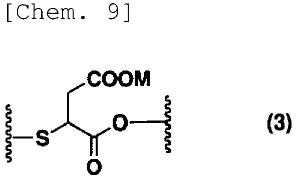 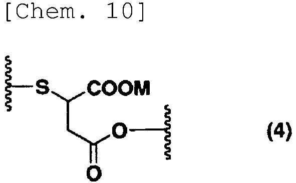 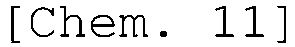 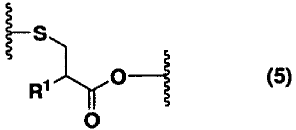
[In general formulas (3) and (4), M represents any one of H, Na, K, NHEt₃, and NHEtiPr₂, and in general formula (5), R¹ represents H or Me], and
the film has an elongation of 400% or more and
a ratio of breaking strength to yield point strength of more than 1.

16. A fiber comprising a polymer compound, wherein
the polymer compound has
a structure in which a polypeptide moiety and
at least one structural unit selected from the group consisting of a polyether structure, a polyester structure, and a polycarbonate structure directly bind to each other by a structure represented by the following general formula (3), (4), or (5) such that the polypeptide moiety binds to S of the following general formula (3), (4), or (5), 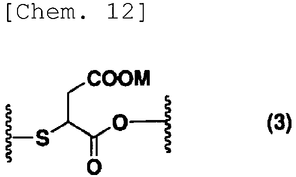 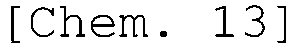 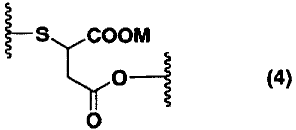 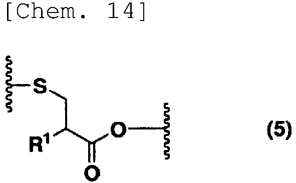
[In general formulas (3) and (4), M represents any one of H, Na, K, NHEt₃, and NHEtiPr₂, and in general formula (5), R¹ represents H or Me], and
the fiber has an elongation of 400% or more and
a ratio of breaking strength to yield point strength of more than 1.

17. A method for producing a solution-state adhesive, the method comprising a step of dissolving a polymer compound obtained by the method according to any one of claims 1 to 4 in a solvent.

18. A method for producing a water-dispersible adhesive, the method comprising a step of dispersing a polymer compound obtained by the method according to any one of claims 1 to 4 in an aqueous medium.

19. A method for producing a film-shaped adhesive, the method comprising a step of molding a film from a solution in which a polymer compound obtained by the method according to any one of claims 1 to 4 is dissolved.

20. A method for producing a powdery adhesive, the method comprising a step of obtaining a powder composition containing a polymer compound obtained by the method according to any one of claims 1 to 4.

21. A method for producing an adhesive body, the method comprising interposing a solution obtained by dissolving a polymer compound obtained by the method according to any one of claims 1 to 4 in a solvent between a plurality of adherends, and then removing the solvent from the solution to solidify the polymer compound, thereby bonding the adherends to each other.

22. A method for producing an adhesive body, the method comprising interposing an aqueous dispersion obtained by dispersing a polymer compound obtained by the method according to any one of claims 1 to 4 in an aqueous medium between a plurality of adherends, and then removing the aqueous medium from the aqueous dispersion to solidify the polymer compound, thereby bonding the adherends to each other.

23. A method for producing an adhesive body, the method comprising softening a film containing a polymer compound obtained by the method according to any one of claims 1 to 4 by swelling or heating, interposing the film between a plurality of adherends, and then curing the film in a state of being brought into pressure contact with the adherends, thereby bonding the adherends to each other.

24. A method for producing an adhesive body, the method comprising heating a powder composition containing a polymer compound obtained by the method according to any one of claims 1 to 4 in a state where the powder composition is interposed between a plurality of adherends, and pressurizing the powder composition via the adherends to solidify the powder composition, thereby bonding the adherends to each other.

25. A method for producing a solution-state coating agent, the method comprising a step of dissolving a polymer compound obtained by the method according to any one of claims 1 to 4 in a solvent.

26. A method for producing a water-dispersible coating agent, the method comprising a step of dispersing a polymer compound obtained by the method according to any one of claims 1 to 4 in an aqueous medium.

27. A method for producing a film-shaped coating agent, the method comprising a step of molding a film from a solution in which a polymer compound obtained by the method according to any one of claims 1 to 4 is dissolved.

28. A method for producing a powdery coating agent, the method comprising a step of obtaining a powder composition containing a polymer compound obtained by the method according to any one of claims 1 to 4.

29. A method for producing a laminate including a base material and a coating layer laminated on at least a part of a surface of the base material, the method comprising
supplying a solution obtained by dissolving a polymer compound obtained by the method according to any one of claims 1 to 4 in a solvent to at least a part of the surface of the base material to coat at least a part of the surface of the base material with the solution, and then removing the solvent from the solution to solidify the polymer compound, thereby laminating the coating layer on at least a part of the surface of the base material.

30. A method for producing a laminate including a base material and a coating layer laminated on at least a part of a surface of the base material, the method comprising
supplying an aqueous dispersion obtained by dispersing a polymer compound obtained by the method according to any one of claims 1 to 4 in an aqueous medium to at least a part of the surface of the base material to coat at least a part of the surface of the base material with the aqueous dispersion, and then removing the aqueous medium from the aqueous dispersion to solidify the polymer compound, thereby laminating the coating layer on at least a part of the surface of the base material.

31. A method for producing a laminate including a base material and a coating layer laminated on at least a part of a surface of the base material, the method comprising
softening a film containing a polymer compound obtained by the method according to any one of claims 1 to 4 by swelling or heating, placing the film on at least a part of the surface of the base material, and then curing the film in a state of being brought into pressure contact with the base material, thereby laminating the coating layer on at least a part of the surface of the base material.

32. A method for producing a laminate including a base material and a coating layer laminated on at least a part of a surface of the base material, the method comprising
heating a powder composition containing a polymer compound obtained by the method according to any one of claims 1 to 4 in a state where the powder composition is placed on at least a part of the surface of the base material, and pressurizing the powder composition between a pressurizing body and the base material to solidify the powder composition, thereby laminating the coating layer on at least a part of the surface of the base material.
